(19) Europäisches Patentamt
European Patent Office
Office européen des brevets

(11) EP 1 224 294 B1

(12) # EUROPEAN PATENT SPECIFICATION

(45) Date of publication and mention of the grant of the patent:
**03.10.2007 Bulletin 2007/40**

(21) Application number: **00971589.7**

(22) Date of filing: **26.10.2000**

(51) Int Cl.:
***C12N 15/53*** *(2006.01)* ***C12N 9/02*** *(2006.01)*
***C12Q 1/66*** *(2006.01)*

(86) International application number:
**PCT/GB2000/004133**

(87) International publication number:
**WO 2001/031028 (03.05.2001 Gazette 2001/18)**

(54) **MUTANT LUCIFERASE**

MUTANTENLUCIFERASE

LUCIFERASE MUTANTE

(84) Designated Contracting States:
**AT BE CH CY DE DK ES FI FR GB GR IE IT LI LU MC NL PT SE**

(30) Priority: **26.10.1999 GB 9925161**
**10.07.2000 GB 0016744**

(43) Date of publication of application:
**24.07.2002 Bulletin 2002/30**

(73) Proprietor: **THE SECRETARY OF STATE FOR DEFENCE**
**Salisbury**
**Wiltshire SP4 OJQ (GB)**

(72) Inventors:
- **SQUIRRELL, David, James**
  **Salisbury**
  **Wiltshire SP4 0JQ (GB)**
- **MURPHY, Melenie, Jane**
  **Salisbury**
  **Wiltshire SP4 0JQ (GB)**
- **PRICE, Rachel, Louise**
  **Salisbury**
  **Wiltshire SP4 0JQ (GB)**
- **WHITE, Peter, John**
  **Salisbury**
  **Wiltshire SP4 0JQ (GB)**
- **WILLEY, Tara, Louise**
  **Department of Bio Chemistry**
  **80 Tennis Court Road**
  **Cambridge CB2 1GA (GB)**

(74) Representative: **Beckham, Robert William et al**
**D/IPR Formalities Section,**
**Poplar 2,**
**MOD Abbey Wood 2218**
**Bristol BS34 8JH (GB)**

(56) References cited:
**EP-B1- 0 751 996** **WO-A-01/20002**
**WO-A-95/25798** **WO-A-99/14336**

- **LI YE ET AL.: "Cloning and sequencing of a cDNA for firefly luciferase from Photuris pennsylvanica" BIOCHIMICA ET BIOPHYSICA ACTA, vol. 1339, 25 April 1997 (1997-04-25), pages 39-52, XP000909154 ISSN: 0006-3002 cited in the application**
- **WHITE P.J. ET AL: "Improved thermostability of the North American firefly luciferase: saturation mutagenesis at position 354" BIOCHEMICAL JOURNAL, vol. 319, 1996, pages 343-350, XP002097112 ISSN: 0264-6021**
- **VIVIANI V.R. ET AL.: "Cloning, sequence analysis and expression of active Phrixothrix railroad-worms luciferases: relationship between bioluminescence spectra and primary structures" BIOCHEMISTRY, vol. 38, no. 26, 29 June 1999 (1999-06-29), pages 8271-8279, XP002172177 cited in the application**
- **LIPMAN D.J. AND PEARSON W.R.: "Rapid and sensitive protein similarity searches" SCIENCE, vol. 227, 22 March 1985 (1985-03-22), pages 1435-1441,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 224 294 B1

SEQUENCE LISTING

<110> The Secretary of State for Defence
White, Peter J
Willey, Tara L
Price, Rachel L
Murphy, Melanie J
Squirrell, David

<120> Novel Enzyme

<130> DERA/IPD/P1247/WOD

<140>
<141>

<150> GB 9925161.3
<151> 1999-10-26

<150> GB 0016744.5
<151> 2000-07-10

<160> 13

<170> PatentIn Ver. 2.1

<210> 1

<211> 1661
<212> DNA
<213> Artificial Sequence

<220>
<221> CDS
<222> (3)..(1661)

<220>
<223> Description of Artificial Sequence: cDNA sequence of the synthetic luciferase gene

<400> 1

```
gg atc caa atg gaa gac gcc aaa aac atc aag aaa ggc ccg gcg cca     47
   Ile Gln Met Glu Asp Ala Lys Asn Ile Lys Lys Gly Pro Ala Pro
        -1   1               5                   10

ttc tat cct ctg gag gat ggc acc gct ggc gag caa ctg cat aag gct    95
Phe Tyr Pro Leu Glu Asp Gly Thr Ala Gly Glu Gln Leu His Lys Ala
     15                  20                  25

atg aag cgt tac gcc ctg gtt cct ggt aca att gct ttt aca gat gca    143
Met Lys Arg Tyr Ala Leu Val Pro Gly Thr Ile Ala Phe Thr Asp Ala
 30                  35                  40                  45

cat atc gag gtg aac atc acg tac gcg gaa tac ttc gaa atg tcc gtt    191
His Ile Glu Val Asn Ile Thr Tyr Ala Glu Tyr Phe Glu Met Ser Val
                 50                  55                  60

cgc ctg gca gaa gct atg aaa cgc tat ggt ctg aat aca aat cac cgt    239
Arg Leu Ala Glu Ala Met Lys Arg Tyr Gly Leu Asn Thr Asn His Arg
             65                  70                  75
```

```
atc gtc gta tgc agt gaa aac tct ctt caa ttc ttt atg ccg gtg ctg    287
Ile Val Val Cys Ser Glu Asn Ser Leu Gln Phe Phe Met Pro Val Leu
        80                  85                  90

ggc gcg ctt ttt atc ggt gtt gca gtt gcg ccg gcg aac gac att tat    335
Gly Ala Leu Phe Ile Gly Val Ala Val Ala Pro Ala Asn Asp Ile Tyr
    95                  100                 105

aat gaa cgt gaa ctg ctt aac agt atg aac att tcg cag cct acc gta    383
Asn Glu Arg Glu Leu Leu Asn Ser Met Asn Ile Ser Gln Pro Thr Val
110                 115                 120                 125

gtc ttt gtt tcc aaa aag ggc ctg caa aaa att ctc aac gtg caa aaa    431
Val Phe Val Ser Lys Lys Gly Leu Gln Lys Ile Leu Asn Val Gln Lys
                130                 135                 140

aaa ctg cca att atc cag aaa att att atc atg gat tct aaa acg gat    479
Lys Leu Pro Ile Ile Gln Lys Ile Ile Ile Met Asp Ser Lys Thr Asp
            145                 150                 155

tac cag ggc ttt cag tcg atg tac acg ttc gtc aca tct cat ctg cct    527
Tyr Gln Gly Phe Gln Ser Met Tyr Thr Phe Val Thr Ser His Leu Pro
        160                 165                 170

ccg ggt ttt aat gaa tac gat ttt gta cca gag tcc ttt gat cgt gac    575
Pro Gly Phe Asn Glu Tyr Asp Phe Val Pro Glu Ser Phe Asp Arg Asp
    175                 180                 185

aaa aca att gca ctg atc atg aat tcc tct ggc tct act ggt ctg cct    623
Lys Thr Ile Ala Leu Ile Met Asn Ser Ser Gly Ser Thr Gly Leu Pro
190                 195                 200                 205

aag ggt gtg gcc ctt ccg cat cgt tgt gcc tgc gtc cgt ttc tcg cat    671
Lys Gly Val Ala Leu Pro His Arg Cys Ala Cys Val Arg Phe Ser His
                210                 215                 220

gcc cgc gat cct att ttt ggt aac caa atc att ccg gat act gcg att    719
Ala Arg Asp Pro Ile Phe Gly Asn Gln Ile Ile Pro Asp Thr Ala Ile
            225                 230                 235

ctg agt gtt gtt cca ttc cac cat ggt ttt ggc atg ttt act aca ctc    767
Leu Ser Val Val Pro Phe His His Gly Phe Gly Met Phe Thr Thr Leu
        240                 245                 250

ggc tat ctg atc tgt ggc ttt cgt gtc gtc ctc atg tat cgc ttt gaa    815
Gly Tyr Leu Ile Cys Gly Phe Arg Val Val Leu Met Tyr Arg Phe Glu
    255                 260                 265

gaa gag ctg ttt ctg cgc tcc ctg cag gat tac aaa att caa agt gcg    863
Glu Glu Leu Phe Leu Arg Ser Leu Gln Asp Tyr Lys Ile Gln Ser Ala
270                 275                 280                 285

ctt ctg gtg cca acc ctg ttt tca ttc ttc gcc aaa agc act ctg att    911
Leu Leu Val Pro Thr Leu Phe Ser Phe Phe Ala Lys Ser Thr Leu Ile
                290                 295                 300

gac aaa tac gat ctg tct aat ctt cac gaa att gct agc ggc ggt gca    959
Asp Lys Tyr Asp Leu Ser Asn Leu His Glu Ile Ala Ser Gly Gly Ala
            305                 310                 315

cct ctt tcg aaa gaa gtc gga gaa gcg gtt gca aaa cgc ttc cat ctt    1007
```

```
Pro Leu Ser Lys Glu Val Gly Glu Ala Val Ala Lys Arg Phe His Leu
        320                 325                 330

cca ggc atc cgt caa ggc tat ggt ctc act gag act act agt gct att    1055
Pro Gly Ile Arg Gln Gly Tyr Gly Leu Thr Glu Thr Thr Ser Ala Ile
    335                 340                 345

ctg att aca ccg aag ggc gat ttc aaa ccg ggc gcg gtc ggt aaa gtg    1103
Leu Ile Thr Pro Lys Gly Asp Phe Lys Pro Gly Ala Val Gly Lys Val
350                 355                 360                 365

gta cca ttt ttt gaa gcg aag gtt gtg gat ctg gat acc ggc aaa acg    1151
Val Pro Phe Phe Glu Ala Lys Val Val Asp Leu Asp Thr Gly Lys Thr
                370                 375                 380

ctg ggc gtt aat cag cgt ggc gaa ctg tgt gtc cgc ggt cct atg att    1199
Leu Gly Val Asn Gln Arg Gly Glu Leu Cys Val Arg Gly Pro Met Ile
            385                 390                 395

atg tcc ggt tat gta aac aat ccg gaa gcg acc aac gcc ctt att gac    1247
Met Ser Gly Tyr Val Asn Asn Pro Glu Ala Thr Asn Ala Leu Ile Asp
        400                 405                 410

aag gat ggc tgg ctg cat tct ggc gac atc gct tac tgg gac gaa gac    1295
Lys Asp Gly Trp Leu His Ser Gly Asp Ile Ala Tyr Trp Asp Glu Asp
    415                 420                 425

gaa cac ttc ttc atc gtt gac cgc ctg aag tct ctc att aaa tac aaa    1343
Glu His Phe Phe Ile Val Asp Arg Leu Lys Ser Leu Ile Lys Tyr Lys
430                 435                 440                 445

ggc tat cag gtg gcc cca gct gaa ctg gaa tcg atc ctc ctg caa cac    1391
Gly Tyr Gln Val Ala Pro Ala Glu Leu Glu Ser Ile Leu Leu Gln His
                450                 455                 460

cca aac atc ttc gac gcg ggc gtg gca ggt ctt ccg gac gat gac gcc    1439
Pro Asn Ile Phe Asp Ala Gly Val Ala Gly Leu Pro Asp Asp Asp Ala
            465                 470                 475

ggt gaa ctt ccg gcc gcc gtt gtt gtt ctc gag cac ggt aag acg atg    1487
Gly Glu Leu Pro Ala Ala Val Val Val Leu Glu His Gly Lys Thr Met
        480                 485                 490

acg gaa aaa gag atc gtg gat tac gtc gcc agt caa gta aca acc gcg    1535
Thr Glu Lys Glu Ile Val Asp Tyr Val Ala Ser Gln Val Thr Thr Ala
    495                 500                 505

aaa aag ctg cgc ggt ggc gtt gtg ttt gtg gac gaa gta ccg aaa ggt    1583
Lys Lys Leu Arg Gly Gly Val Val Phe Val Asp Glu Val Pro Lys Gly
510                 515                 520                 525

ctt acc ggc aaa ctc gac gca cgt aaa atc cgc gag atc ctc att aag    1631
Leu Thr Gly Lys Leu Asp Ala Arg Lys Ile Arg Glu Ile Leu Ile Lys
                530                 535                 540

gcc aag aag ggc ggt aag tcc aag ctt taa                            1661
Ala Lys Lys Gly Gly Lys Ser Lys Leu
            545                 550
```

<210> 2
<211> 552
<212> PRT
<213> Artificial Sequence

<220>
<223> Description of Artificial Sequence: Encoded amino acid sequence of the cDNA sequence of synthetic luciferase gene

<400> 2

```
Ile Gln Met Glu Asp Ala Lys Asn Ile Lys Lys Gly Pro Ala Pro Phe
    -1   1               5                  10
Tyr Pro Leu Glu Asp Gly Thr Ala Gly Glu Gln Leu His Lys Ala Met
15                  20                  25                  30
Lys Arg Tyr Ala Leu Val Pro Gly Thr Ile Ala Phe Thr Asp Ala His
                35                  40                  45
Ile Glu Val Asn Ile Thr Tyr Ala Glu Tyr Phe Glu Met Ser Val Arg
            50                  55                  60
Leu Ala Glu Ala Met Lys Arg Tyr Gly Leu Asn Thr Asn His Arg Ile
        65                  70                  75
Val Val Cys Ser Glu Asn Ser Leu Gln Phe Phe Met Pro Val Leu Gly
    80                  85                  90
Ala Leu Phe Ile Gly Val Ala Val Ala Pro Ala Asn Asp Ile Tyr Asn
95                  100                 105                 110
Glu Arg Glu Leu Leu Asn Ser Met Asn Ile Ser Gln Pro Thr Val Val
                115                 120                 125
Phe Val Ser Lys Lys Gly Leu Gln Lys Ile Leu Asn Val Gln Lys Lys
            130                 135                 140
Leu Pro Ile Ile Gln Lys Ile Ile Ile Met Asp Ser Lys Thr Asp Tyr
        145                 150                 155
Gln Gly Phe Gln Ser Met Tyr Thr Phe Val Thr Ser His Leu Pro Pro
    160                 165                 170
Gly Phe Asn Glu Tyr Asp Phe Val Pro Glu Ser Phe Asp Arg Asp Lys
175                 180                 185                 190
Thr Ile Ala Leu Ile Met Asn Ser Ser Gly Ser Thr Gly Leu Pro Lys
                195                 200                 205
Gly Val Ala Leu Pro His Arg Cys Ala Cys Val Arg Phe Ser His Ala
            210                 215                 220
Arg Asp Pro Ile Phe Gly Asn Gln Ile Ile Pro Asp Thr Ala Ile Leu
        225                 230                 235
Ser Val Val Pro Phe His His Gly Phe Gly Met Phe Thr Thr Leu Gly
    240                 245                 250
Tyr Leu Ile Cys Gly Phe Arg Val Val Leu Met Tyr Arg Phe Glu Glu
255                 260                 265                 270
Glu Leu Phe Leu Arg Ser Leu Gln Asp Tyr Lys Ile Gln Ser Ala Leu
                275                 280                 285
Leu Val Pro Thr Leu Phe Ser Phe Phe Ala Lys Ser Thr Leu Ile Asp
            290                 295                 300
Lys Tyr Asp Leu Ser Asn Leu His Glu Ile Ala Ser Gly Gly Ala Pro
        305                 310                 315
Leu Ser Lys Glu Val Gly Glu Ala Val Ala Lys Arg Phe His Leu Pro
    320                 325                 330
Gly Ile Arg Gln Gly Tyr Gly Leu Thr Glu Thr Thr Ser Ala Ile Leu
335                 340                 345                 350
Ile Thr Pro Lys Gly Asp Phe Lys Pro Gly Ala Val Gly Lys Val Val
                355                 360                 365
Pro Phe Phe Glu Ala Lys Val Val Asp Leu Asp Thr Gly Lys Thr Leu
            370                 375                 380
Gly Val Asn Gln Arg Gly Glu Leu Cys Val Arg Gly Pro Met Ile Met
        385                 390                 395
```

```
Ser Gly Tyr Val Asn Asn Pro Glu Ala Thr Asn Ala Leu Ile Asp Lys
    400                 405                 410
Asp Gly Trp Leu His Ser Gly Asp Ile Ala Tyr Trp Asp Glu Asp Glu
415                 420                 425                 430
His Phe Phe Ile Val Asp Arg Leu Lys Ser Leu Ile Lys Tyr Lys Gly
                435                 440                 445
Tyr Gln Val Ala Pro Ala Glu Leu Glu Ser Ile Leu Leu Gln His Pro
            450                 455                 460
Asn Ile Phe Asp Ala Gly Val Ala Gly Leu Pro Asp Asp Asp Ala Gly
        465                 470                 475
Glu Leu Pro Ala Ala Val Val Val Leu Glu His Gly Lys Thr Met Thr
    480                 485                 490
Glu Lys Glu Ile Val Asp Tyr Val Ala Ser Gln Val Thr Thr Ala Lys
495                 500                 505                 510
Lys Leu Arg Gly Gly Val Val Phe Val Asp Glu Val Pro Lys Gly Leu
                515                 520                 525
Thr Gly Lys Leu Asp Ala Arg Lys Ile Arg Glu Ile Leu Ile Lys Ala
            530                 535                 540
Lys Lys Gly Gly Lys Ser Lys Leu
        545                 550
```

<210> 3
<211> 1661
<212> DNA
<213> Artificial Sequence

<220>
<223> Description of Artificial Sequence: cDNA sequence of synthetic luciferase gene

<400> 3

ttaaagcttg gacttaccgc ccttcttggc cttaatgagg atctcgcgga ttttacgtgc 60
gtcgagtttg ccggtaagac ctttcggtac ttcgtccaca aacacaacgc caccgcgcag 120
ctttttcgcg gttgttactt gactggcgac gtaatccacg atctcttttt ccgtcatcgt 180
cttaccgtgc tcgagaacaa caacggcggc cggaagttca ccggcgtcat cgtccggaag 240
acctgccacg cccgcgtcga agatgtttgg gtgttgcagg aggatcgatt ccagttcagc 300
tggggccacc tgatagcctt tgtatttaat gagagacttc aggcggtcaa cgatgaagaa 360
gtgttcgtct tcgtcccagt aagcgatgtc gccagaatgc agccagccat ccttgtcaat 420
aagggcgttg gtcgcttccg gattgtttac ataaccggac ataatcatag gaccgcggac 480
acacagttcg ccacgctgat taacgcccag cgttttgccg gtatccagat ccacaacctt 540
cgcttcaaaa aatggtacca ctttaccgac cgcgcccggt ttgaaatcgc ccttcggtgt 600
aatcagaata gcactagtag tctcagtgag accatagcct tgacggatgc ctggaagatg 660
gaagcgtttt gcaaccgctt ctccgacttc tttcgaaaga ggtgcaccgc cgctagcaat 720
ttcgtgaaga ttagacagat cgtatttgtc aatcagagtg cttttggcga agaatgaaaa 780
cagggttggc accagaagcg cactttgaat tttgtaatcc tgcagggagc gcagaaacag 840
ctcttcttca aagcgataca tgaggacgac acgaaagcca cagatcagat agccgagtgt 900
agtaaacatg ccaaaaccat ggtggaatgg aacaacactc agaatcgcag tatccggaat 960
gatttggtta ccaaaaatag gatcgcgggc atgcgagaaa cggacgcagg cacaacgatg 1020
cggaagggcc acacccttag gcagaccagt agagccagag gaattcatga tcagtgcaat 1080
tgttttgtca cgatcaaagg actctggtac aaaatcgtat tcattaaaac ccggaggcag 1140
atgagatgtg acgaacgtgt acatcgactg aaagccctgg taatccgttt tagaatccat 1200
gataataatt ttctggataa ttggcagttt tttttgcacg ttgagaattt tttgcaggcc 1260
ctttttggaa acaaagacta cggtaggctg cgaaatgttc atactgttaa gcagttcacg 1320
ttcattataa atgtcgttcg ccggcgcaac tgcaacaccg ataaaaagcg cgcccagcac 1380
cggcataaag aattgaagag agttttcact gcatacgacg atacggtgat ttgtattcag 1440
accatagcgt ttcatagctt ctgccaggcg aacggacatt tcgaagtatt ccgcgtacgt 1500
gatgttcacc tcgatatgtg catctgtaaa agcaattgta ccaggaacca gggcgtaacg 1560
cttcatagcc ttatgcagtt gctcgccagc ggtgccatcc tccagaggat agaatggcgc 1620
cgggcctttc ttgatgtttt tggcgtcttc catttggatc c 1661

<210> 4
<211> 30
<212> DNA
<213> Artificial Sequence

<220>
<223> Description of Artificial Sequence: Oligonucleotide

<400> 4
cacccgaggg ggattataaa ccgggcgcgg 30

<210> 5
<211> 34
<212> DNA
<213> Artificial Sequence

<220>
<223> Description of Artificial Sequence: Oligonucleotide

<400> 5
cacccgaggg ggatvycaaa ccgggcgcgg tcgg 34

<210> 6
<211> 34
<212> DNA
<213> Artificial Sequence

<220>
<223> Description of Artificial Sequence: Oligonucleotide

<400> 6
cacccgaggg ggattdsaaa ccgggcgcgg tcgg 34

<210> 7
<211> 34
<212> DNA
<213> Artificial Sequence

<220>
<223> Description of Artificial Sequence: Oligonucleotide

<400> 7
cacccgaggg ggatmrsaaa ccgggcgcgg tcgg 34

<210> 8
<211> 40
<212> DNA
<213> Artificial Sequence

<220>
<223> Description of Artificial Sequence:
Oligonucleotide

<400> 8
gggctcactg agactactag tgctattatg attacacccg 40

<210> 9
<211> 40
<212> DNA
<213> Artificial Sequence

<220>
<223> Description of Artificial Sequence: Oligonucleotide

<400> 9
cgggtgtaat cagaatagca ctagtagtct cagtgagccc 40

<210> 10
<211> 36
<212> DNA
<213> Artificial Sequence

<220>
<223> Description of Artificial Sequence:
Oligonucleotide

<400> 10
ggcgcggtcg gtaaagtggt accatttttt gaagcg 36

<210> 11
<211> 36
<212> DNA
<213> Artificial Sequence

<220>

<223> Description of Artificial Sequence:
Oligonucleotide

<400> 11
cgcttcaaaa aatggtacca ctttaccgac cgcgcc 36

<210> 12
<211> 62
<212> DNA
<213> Artificial Sequence

<220>
<221> misc_feature
<222> (24)..(25)
<223> n=a or g or c or t

<220>
<221> misc_feature
<222> (33)..(34)
<223> n=a or g or c or t

<220>
<223> Description of Artificial Sequence:
Oligonucleotide

<400> 12

ctagtgctat tctgattaca cccnnsgggg atnnsaaacc gggcgcggtc ggtaaagtgg 60
ta 62

<210> 13
<211> 55
<212> DNA
<213> Artificial Sequence

<220>
<221> misc_feature
<222> (26)..(27)
<223> n=a or g or c or t

<220>
<221> misc feature
<222> (35)..(36)
<223> n=a or g or c or t

<220>
<223> Description of Artificial Sequence: Oligonucleotide

<400> 13
cactttaccg accgcgcccg gtttsnnatc cccsnngggt gtaatcagaa tagca 55

## Description

The present invention relates to a novel protein, in particular mutant luciferase enzymes which show distinctive properties as compared to corresponding wild type enzyme, to DNA encoding these proteins, to the use of these enzyme in assays and to test kits containing them.

Firefly luciferase catalyses the oxidation of luciferin in the presence of ATP, $Mg^{2+}$ and molecular oxygen with the resultant production of light. This reaction has a quantum yield of about 0.88. The light emitting property has led to its use in a wide variety of luminometric assays where ATP levels are being measured. Examples of such assays include those which are based upon the described in EP-B-680515 and WO 96/02665 but many others are used routinely in laboratories.

Luciferase is obtainable directly from the bodies of insects, in particular beetles such as fireflies or glow-worms. Particular species from which luciferases have been obtained include the Japanese GENJI or KEIKE fireflies, *Luciola cruciata* and *Luciola lateralis*, the East European firefly *Luciola mingrelica*, the North American firefly *Photinus pyralis* and the glow-worm *Lampyris noctiluca.*

However, since many of the genes encoding these enzymes have been cloned and sequenced, they may also be produced using recombinant DNA technology. Recombinant DNA sequences encoding the enzymes are used to transform microorganisms such as *E. coli* which then express the desired enzyme product.

The colour of the light emitted by these enzymes when used in assays in the laboratory are broadly similar. It would be helpful if the wavelength could be altered, either to be more easily read by the specific detector, or for use in systems where multiple reporters are required, for example to monitor different events within the same sample. One way of distinguishing reporter molecules is to utilise luciferase molecules which emit light at distinct wavelengths. This may be achieved by using reporter molecules comprising luciferases derived from different species of beetle or glow-worm. An alternative strategy however is to produce mutant luciferases using recombinant DNA technology, so as to produce a variation in the wavelength of the signal. Examples of such mutants are provided in WO 95/18853.

Furthermore, the heat stability of wild and recombinant type luciferases is such that they lose activity quite rapidly when exposed to temperatures in excess of about 30°C, particularly over 35°C. This instability causes problems when the enzyme is used or stored at high ambient temperature, or if the assay is effected under high temperature reaction conditions, for example in order to increase reaction rate.

Mutant luciferases having increased thermostability are known from EP-A-524448 and WO/95/25798. The first of these describes a mutant luciferase having a mutation at position 217 in the Japanese firefly luciferase, in particular by replacing a threonine residue with an isoleucine residue. The latter describes mutant luciferases having over 60% similarity to luciferase from *Photinus pyralis, Luciola mingrelica, Luciola cruciata* or *Luciola lateralis* but in which the amino acid residue corresponding to residue 354 of *Photinus pyralis* or 356 of the *Luciola* species is mutated such that it is other than glutamate, and in particular is other than glutamate, aspartate, proline or glycine.

Co-pending British Patent GB 2345913 and the International Patent Application derived from it WO0024878, describes further such mutants. In this case, proteins are described which have luciferase activity and at least 60% similarity to wild-type luciferase such as those from *Photinus pyralis, Luciola mingrelica, Luciola cruciata* or *Luciola lateralis* enzyme, but which include mutations at various positions in the protein, including amongst others, (a) the amino acid residue corresponding to residue 214 in *Photinus pyralis* luciferase and to residue 216 of *Luciola mingrelica, Luciola cruciata* or *Luciola lateralis* luciferase; or

(b) the amino acid residue corresponding to residue 232 in *Photinus pyralis* luciferase and to residue 234 of *Luciola mingrelica, Luciola cruciata* or *Luciola lateralis* luciferase; or

(c) amino acid residue corresponding to residue 295 in *Photinus pyralis* luciferase and to residue 297 of *Luciola mingrelica, Luciola cruciata* or *Luciola lateralis* luciferase.

WO 01/20002 describes the use of directed evolution to find mutant luciferases having multiple mutations, which have improved properties.

The applicants have found that by mutating (or introducing) an amino acid at a different position within the luciferase protein, large shifts in the wavelength of the emitted light may be achieved and/or the enzyme has improved thermostability. Furthermore, the proton flux of emitted light may be improved, making the enzyme better suited to *in vivo* assays where glow kinetics are precluded or in vitro assays where CoA or other 'glow kinetic inducing' compounds are not present.

The present invention provides a recombinant protein having luciferase activity and at least 90% similarity to a wild-type luciferase from *Photinus pyralis, Luciola mingrelica, Luciola cruciata* or *Luciola lateralis, Hotaria parvula, Pyxophorus plagiophthalamus, Lampyris noctiluca, Pyrocoelia miyako,* or *Phrixothrix* (railroad-worms - see Biochem. 38 (1999) 8271-8279) wherein in the sequence of the enzyme, the amino acid residue corresponding to residue 357 in *Photinus pyralis* luciferase is mutated as compared to the corresponding wild-type luciferase, such that the luciferase enzyme is able to emit light at a different wavelength as compared to the corresponding wild-type luciferase and/or has enhanced thermostability as compared to the corresponding wild-type luciferase, and wherein similarity is determined using an alignment algorithm as defined by Lipman and Pearson (1985, Science 227: 1435-1441) with the parameters ktup = 1, gap penalty = 4 and gap penalty length = 12.

Bioluminescent enzymes from species that can use the substrate D-luciferin (4,5-dihydro-2-[6-hydroxy-2-benzothiazolyl]-4-thiazole carboxylic acid) to produce light emission may form the basis of the mutant enzymes of the invention.

In particular, the luciferases are enzymes obtainable from *Photinus pyralis*, *Luciola mingrelica*, *Luciola cruciata* or *Luciola lateralis* enzyme. In *Luciola mingrelica*, *Luciola cruciata* or *Luciola lateralis* enzymes, the appropriate amino acid residue is at position 359 in the sequence.

The sequences of all the various luciferases show that they are highly conserved having a significant degree of similarity between them. This means that corresponding regions among the enzyme sequences are readily determinable by examination of the sequences to detect the most similar regions. Corresponding acids can be determined by reference to L. Ye et al., Biochim. Biophys Acta 1339 (1997) 39-52 which shows the sequences of the enzymes, together with the numbering, which numbering system is to be used in connection with the present application.

As regards the possible change of the amino acid residue corresponding to residue 357 in *Photinus pyralis* luciferase, most wild-type sequences have an acid residue (aspartic acid or glutamic acid) at this position. The exception to this is some forms of the luciferase of *Photuris pennsylvanica* in which the corresponding residue (356) is the non-polar residue, valine or some forms of *Phrixothrix* luciferase where the corresponding position is V354 in $Pv_{GR}$ or in $Ph_{RE}$, where it is L355 leucine. Thus in general, the amino acid used as a substitute amino acid at this position is other than aspartic acid, glutamic acid, valine or leucine.

In most cases, therefore, an acidic amino acid residue is replaced with a non-acidic residue, including basic amino acids such as lysine or arginine, non-polar amino acids such as leucine, valine or isoleucine, uncharged polar amino acids such as tyrosine, asparagine, glutamine, phenylalanine, serine, tryptophan or threonine. In particular, it may be replaced with an uncharged polar amino acid such as tyrosine, asparagine, serine or threonine. Particularly preferred amino acid residues for substitution at this position are tyrosine, phenylalanine or tryptophan and most preferably tyrosine. Generally speaking, aromatic residues at this position give rise to the largest shifts and may also assist thermostability.

Where wild-type sequences include non-acidic amino acid residues at this position, they are suitably mutated into different non-acidic residues.

It has been found that by mutating the enzyme in this way, the wavelength of light emitted by the luciferase is shifted, in some cases up to 50nm towards the red end of the spectrum. Thus, D357Y mutant Photinus pyralis luciferase emits light at a wavelength of some 612nm as compared to the wild-type enzyme which emits light at a wavelength of 562nm.

A wavelength shift of 50nm has considerable potential for use in assay applications as a shift of this magnitude can be readily defined spectrally. Different coloured luciferases could be employed as reporter molecules in gene expression studies, enabling the simultaneous monitoring of more than one gene, for example as described in WO 95/18853. Multiple analyte testing could also be performed with luciferase as labels.

The fact that the light in this case is a deep red in colour is particularly useful in assay methodology. A red mutant could be useful when analysing a solution for ATP which contained pigments or other compounds which may absorb shorter wavelengths of light. For example, a red coloured solution would not absorb red light. Examples of red coloured solutions which are frequently the subject of such analysis include blood samples or a solution of eukaryotic cell culture medium which may contain a red coloured pH indicator.

When using a mixture of colourimetric agents such as luciferases, the ability to generate a deep red signal may be helpful, particularly where another agent in the sample generates a green signal. A photomultiplier tube used in photocathode spectral analysis can be set to detect either one or both peaks generated in a single sample. In other words, it is possible to distinguish between photon flux from a red and green emitter in the same sample.

Furthermore, it has been found that the wavelength shift can be affected by the presence of the cofactor coenzyme A (CoA). This feature gives rise to the possibility that this enzyme could be used in an assay for the cofactor.

As described below, the effect the cofactor coenzyme A on the in vitro spectrum of emitted light was investigated. As the concentration of coenzyme A increases the spectral distribution alters and at the highest concentrations of CoA the spectrum is dominated by wavelengths in the region 590-630nm with a pronounced peak at 610nm.

Thus in accordance with a further aspect of the invention, there is provided an assay for determining the presence in a sample of CoA, which assay comprises adding to a sample suspected of containing CoA, luciferase as described above together with other reagents which are required to bring about a luciferase/luciferin reaction, measuring the wavelength of light emitted from the sample and relating this to the presence or absence of CoA.

Such an assay may be useful in the detection of the state of growth or activity of cells, for example microorganisms or eukaryotic cells.

For example, the concentration of CoA in E. *coli* cells is relatively high, and varies considerably with metabolic status. The mutant enzymes of the invention can be used to monitor the metabolic status of an organism, particularly the *in vivo* concentration of the CoA, since the wavelength of the emission varies depending upon the CoA concentration. Such assays may be particularly useful in situations where CoA is an important primary metabolite in the production of antibiotics (e.g. in streptomycetes). Cellular CoA concentrations are also an important indicator of fatty acid biosynthesis and vary with the starvation status of the cell. A number of metabolic disorders such as carcinogenesis and diabetes, show abnormalities in the fatty acid metabolites and consequently unusual CoA levels. Assays of the invention may be used in the diagnosis of such conditions. For example, the CoA levels from within a cell sample, such as a blood sample, from a patient, may be determined by measuring the wavelength of light emitted from a luciferase of the invention, used in the assay. This result may be compared with that obtained from a sample of healthy cells to determine whether the wavelength has changed and thus that a modified CoA level is present. This may be indicative of a disease state in the patient. Cells are suitably lysed prior to assay using a known lytic agent.

It is believed that the amino acid residue at position 357 is critically associated with the binding site of coenzyme A. When the surface of the luciferase enzyme was contoured (using SYBL protein modelling software, Tripos Ltd.) to a resolution of 1 Angstrom (Å), a small polar pocket was noted. This pocket appears to be lined by residues H310, E354 and D357 and measured between 8-10Å. When viewed from the top of the molecule, this pocket appears as part of a larger pocket, lined by residues H310, E354, D357 and I232. Residues H310 and E354 appear to form a bridge across the cleft giving the appearance of two smaller pockets (See Figure 8).

Without being bound by theory, it seems possible that the bridging residues may be flexible enough to disengage when the enzyme is in solution to provide a larger pocket (~12Å deep and ~8Å wide) which allows CoA binding. This is consistent with the energy calculations.

When *E. coli* cells expressing mutants of firefly luciferase of the invention were grown on different carbon sources changes in the *in vivo* spectrum of emitted light were observed. Switching from a rich medium (LB) to a defined minimal medium with either acetate or glucose as the sole carbon source resulted in shifts to longer wavelengths of emitted light and a reduction in the contribution from shorter wavelengths. This may provide yet a further means of controlling the wavelength of light emitted for assay purposes.

Mutation of the 357 position of in the protein has been found to result in enhanced thermostability.

The proteins may contain further mutations in the sequence provided the luciferase activity of the protein is not unduly compromised. The mutations suitably enhance the properties of the enzyme or better suit it for the intended purpose in some way. This may mean that they result in enhanced thermostability and/or colour shift properties, and/or the $K_m$ for ATP of the enzymes. Examples of mutations which give rise to colour shifts are described in WO95/18853. Mutations which affect $K_m$values are described for example in WO 96/22376 and International Patent Application No. WO9846729.

In general, effects of mutations have been found to be additive in terms of alterations in the properties.

The mutant luciferases of the invention may include other specific mutations which enhance thermostability as compared to wild-type luciferase. In particular, at least one of

(a) the amino acid residue corresponding to amino acid 354 of the *Photinus pyralis* luciferase (356 in *Luciola* luciferase) is mutated;

(b) the amino acid residue corresponding to position 215 in *Photinus pyralis* luciferase or (217 in *Luciola* luciferase) is a different hydrophobic amino acid; or

(c) the amino acid residue corresponding to residue 214 in *Photinus pyralis* luciferase or to residue 216 of *Luciola mingrelica*, *Luciola cruciata* or *Luciola lateralis* luciferase;

(d) the amino acid residue corresponding to residue 232 in *Photinus pyralis* luciferase or to residue 234 of *Luciola mingrelica, Luciola cruciata* or *Luciola lateralis* luciferase;

(e) amino acid residue corresponding to residue 295 in *Photinus pyralis* luciferase or to residue 297 of *Luciola mingrelica, Luciola cruciata* or *Luciola lateralis* luciferase;

(f) amino acid residue corresponding to amino acid 14 of the *Photinus pyralis* luciferase or to residue 16 of *Luciola mingrelica*, or 17 in *Luciola cruciata* or *Luciola lateralis*;

(g) amino acid residue corresponding to amino acid 35 of the *Photinus pyralis* luciferase or to residue 37 of *Luciola mingrelica,* or to residue 38 of *Luciola cruciata* or *Luciola lateralis;*

(h) amino acid residue corresponding to amino acid residue 105 of the *Photinus pyralis* luciferase or to residue 106 of *Luciola mingrelica*, 107 of *Luciola cruciata or Luciola lateralis*;

(i) amino acid residue corresponding to amino acid residue 234 of the *Photinus pyralis* luciferase or to residue 236 of *Luciola mingrelica, Luciola cruciata* or *Luciola lateralis;*

(j) amino acid residue corresponding to amino acid residue 420 of the *Photinus pyralis* luciferase or to residue 422 of *Luciola mingrelica, Luciola cruciata* or *Luciola lateralis;*

(k) amino acid residue corresponding to amino acid residue 310 of the *Photinus pyralis* luciferase or to residue 312 of *Luciola mingrelica*, *Luciola cruciata* or *Luciola lateralis;*

is different to the amino acid which appears in the corresponding wild type sequence and wherein the luciferase enzyme has increased thermostability as compared to an enzyme having the amino acid of the corresponding wild-type luciferase at this position.

Thus preferred examples of proteins of the invention are mutated wild-type luciferases where more than one amino acid, for example up to 100 amino acid residues, preferably no more than 40 amino acids, and more preferably up to 30 amino acids, are different to the amino acid at the corresponding position in the appropriate wild-type enzyme.

Thus, in one preferred embodiment, the protein of the invention comprises luciferase of *Photinus pyralis,* wherein, in addition to the mutation at the 357 position as described above, at least one of;

(a) the amino acid residue corresponding to amino acid 354 of the *Photinus pyralis* luciferase is other than glutamate;
(b) the amino acid residue corresponding to position 215 in *Photinus pyralis* luciferase or is a hydrophobic amino acid other than alanine;
(c) the amino acid residue corresponding to residue 214 in *Photinus pyralis* luciferase is other than threonine;
(d) the amino acid residue corresponding to residue 232 in *Photinus pyralis* luciferase is other than isoleucine;
(e) amino acid residue corresponding to residue 295 in *Photinus pyralis* luciferase is other than phenylalanine;
(f) amino acid residue corresponding to amino acid 14 of the *Photinus pyralis* luciferase is other than phenylalanine;
(g) amino acid residue corresponding to amino acid 35 of the *Photinus pyralis* luciferase is other than leucine;
(h) amino acid residue corresponding to amino acid residue 105 of the *Photinus pyralis* luciferase is other than alanine;
(i) amino acid residue corresponding to amino acid residue 234 of the *Photinus pyralis* luciferase is other than aspartic acid;
(j) amino acid residue corresponding to amino acid residue 420 of the *Photinus pyralis* luciferase is other than serine;
(k) amino acid residue corresponding to amino acid residue 310 of the *Photinus pyralis* luciferase is other than histidine.

Alternatively, the protein of the invention comprises protein the luciferase sequence of *Luciola mingrelica, Luciola cruciata* or *Luciola lateralis* enzyme, and wherein, in addition to the mutation at position 359 as described above, at least one of

(a) the amino acid residue corresponding to amino acid 356 of the *Photinus pyralis* luciferase is other than glutamate;
(b) the amino acid residue corresponding to position 215 in *Photinus pyralis* luciferase or is a hydrophobic amino acid other than alanine or threonine;
(c) the amino acid residue corresponding to residue 216 of *Luciola mingrelica, Luciola cruciata* or *Luciola lateralis* luciferase is other than glycine (for *Luciola mingrelica* based sequences) or aparagine (for *Luciola cruciata* or *Luciola lateralis)* based sequences;
(d) the amino acid residue corresponding to residue 234 of *Luciola mingrelica, Luciola cruciata* or *Luciola lateralis* luciferase is other than serine;
(e) amino acid residue corresponding to residue 297 of *Luciola mingrelica, Luciola cruciata* or *Luciola lateralis* luciferase is other than leucine;
(f) amino acid residue corresponding to amino acid 16 of *Luciola mingrelica,* of amino acid 17 of *Luciola cruciata* or *Luciola lateralis* is other than phenylalanine;
(g) amino acid residue corresponding to residue 37 of *Luciola mingrelica,* or 38 in *Luciola cruciata* or *Luciola lateralis* is other than lysine;
(h) amino acid residue corresponding to amino acid residue 106 of *Luciola mingrelica,* or amino acid residue 107 of *Luciola cruciata* or *Luciola lateralis* is other than glycine;
(i) amino acid residue corresponding to amino acid residue 236 of *Luciola mingrelica*, *Luciola cruciata* or *Luciola lateralis* is other than glycine;
(j) amino acid residue corresponding to residue 422 of *Luciola mingrelica*, *Luciola cruciata* or *Luciola lateralis* is other than threonine;
(k) amino acid residue corresponding to amino acid residue 312 of *Luciola mingrelica*, *Luciola cruciata* or *Luciola lateralis* is other than threonine (for *Luciola mingrelica* based sequences) or valine (for *Luciola cruciata* or *Luciola lateralis*) based sequences.

The particular substituted amino acids in any case which give rise to enhanced thermostability can be determined by routine methods as illustrated hereinafter. In each case, different substitutions may result in enhanced thermostability. Substitution may be effected by site-directed mutagenesis of DNA encoding native or suitable mutant proteins as would be understood by the skilled person. The invention in this case is associated with the identification of the positions which are associated with thermostability.

In general however, it may be desirable to consider substituting an amino acid of different properties for the wild type amino acid. Thus hydrophilic amino acid residues may, in some cases be preferably substituted with hydrophobic amino acid residues and vice versa. Similarly, acidic amino acid residues may be substituted with basic residues.

For instance, the protein may comprise a protein having luciferase activity and at least 60% similarity to luciferase from *Photinus pyralis*, *Luciola mingrelica*, *Luciola cruciata* or *Luciola lateralis* enzyme wherein in the sequence of the enzyme, at least one of;

(a) the amino acid residue corresponding to residue 211 in Photinus *pyralis* luciferase and to residue 216 of *Luciola mingrelica*, *Luciola cruciata* or *Luciola lateralis* luciferase is mutated and is other than threonine in the case of *Photinus pyralis* luciferase; or
(b) the amino acid residue corresponding to residue 232 in *Photinus pyralis* luciferase and to residue 234 of *Luciola mingrelica, Luciola cruciata* or *Luciola lateralis* luciferase is mutated and is other than isoleucine in the case of *Photinus pyralis* luciferase; or
(c) amino acid residue corresponding to residue 295 in *Photinus pyralis* luciferase and to residue 297 of *Luciola mingrelica, Luciola cruciata* or *Luciola lateralis* luciferase is mutated and is for example, other than phenylalanine in the case of *Photinus pyralis* luciferase;

and the luciferase enzyme has increased thermostability as compared to the wild-type luciferase.

With respect to the possible change of the amino acid residue corresponding to residue 214 in *Photinus pyralis* luciferase, the polar amino acid threonine is suitably replaced with a non polar amino acid such as alanine, glycine, valine, leucine, isoleucine, proline, phenylalanine, methionine, tryptophan or cysteine. A particularly preferred substitution for the threonine residue corresponding to residue 214 in *Photinus pyralis* is alanine. A more preferred substitution is cysteine. However, different polar residues such as asparagine at this position may also enhance the thermostability of the corresponding enzyme having threonine at this position. Other amino acids which appear at this position in wild-type luciferase enzymes include glycine *(Luciola mingrelica, Hotaria parvula),* asparagine *(Pyrophorus plagioph thalamus, GR, YG*, *YE and OR, Luciola cruciata, Luciola lateralis, Lampyris noctiluca, Pyrocelia miyako Photuris pennsylvanica LY, KW*, *J19)* and serine (*Phrixothix*). These may advantageously be substituted with non-polar or different non-polar side chains such as alanine and cysteine.

As regards the possible change of the amino acid residue corresponding to residue 232 in *Photinus pyralis* luciferase, the nonpolar amino acid isoleucine is suitably replaced with a different non polar amino acid such as alanine, glycine, valine, lecine, proline, phenylalanine, methionine, tryptophan or cysteine. Other amino acids appearing at this position in wild type sequences include serine and asparagine. Suitably, these polar residues are substituted by non-polar residues such as those outlined above. A particularly preferred substitution for the residue corresponding to residue 232 in *Photinus pyralis* luciferase and to residue 234 of *Luciola mingrelica*, *Luciola cruciata* or *Luciola lateralis* luciferase at group is alanine.

Changes of the amino acid residue corresponding to residue 295 in *Photinus pyralis* luciferase and to residue 297 of *Luciola mingrelica, Luciola cruciata* or *Luciola lateralis luciferase,* may also affect the thermostability of the protein. (This corresponds to position 292 in *Phrixothix* luciferase.) In general, the amino acid at this position is a non-polar amino acid phenylalanine or leucine. These are suitably changed for different non-polar amino acids. For example, in *Photinus pyralis,* the non-polar amino acid phenylalanine is suitably replaced with a different non polar amino acid, such as alanine, leucine, glycine, valine, isoleucine, proline, methionine, tryptophan or cysteine. A particularly preferred substitution for the phenylalanine residue corresponding to residue 214 in *Photinus pyralis* luciferase is leucine.

Mutation at the amino acid residue corresponding to amino acid 14 of the *Photinus pyralis* luciferase or to amino acid 16 or 17 in Luciola luciferase (13 in *Phrixothrix* luciferase) is also possible. This amino acid residue (which is usually phenylalanine, but may also be leucine, serine, arginine or in some instances tyrosine) is suitably changed to a different amino acid, in particular to a different nonpolar amino acid such as alanine, valine, leucine, isoleucine, proline, methionine or tryptophan, preferably alanine.

Mutation at the amino acid residue corresponding to amino acid 35 of the *Photinus pyralis* luciferase or to amino acid residue 37 in Luciola mingrelica luciferase (38 in other *Luciola* spp.) may also be effective. This amino acid varies amongst wild type enzymes, which may include leucine (*Photinus pyralis*) but also lysine, histidine, glycine, alanine, glutamine and aspartic acid at this position. Suitably the amino residue at this position is substituted with a non-polar amino acid residue or a different non-polar amino acid such as alanine, valine, phenylalanine, isoleucine, proline, methionine or tryptophan. A preferred amino acid at this position is alanine, where this is different to the wild-type enzyme.

Mutations at the amino acid corresponding to position 14 of the *Photinus pyralis* sequence and/or mutation at the amino acid residue corresponding to amino acid 35 of the *Photinus pyralis* luciferase are preferably not the only mutation in the enzyme.

They are suitably accompanied by others of the mutations defined above, in particular those at positions corresponding to positions 214, 395 or 232 of *Photinus pyralis* luciferase.

Changes of the amino acid residue corresponding to residue 105 in *Photinus pyralis* luciferase and to residue 106 of *Luciola mingrelica*, or residue 107 of *Luciola cruciata* or *Luciola lateralis* luciferase, (102 in *Phrixothrix*) may also affect the thermostability of the protein. In general, the amino acid at this position is a non-polar amino acid alanine or glycine, or serine in *Phrixothrix.* These are suitably changed for different non-polar amino acids. For example, in *Photinus pyralis,* the non-polar amino acid alanine is suitably replaced with a different non polar amino acid, such as phenylalanine, leucine, glycine, valine, isoleucine, proline, methionine or tryptophan. A particularly preferred substitution for the alanine residue corresponding to residue 105 in *Photinus pyralis* luciferase is valine.

Changes of the amino acid residue corresponding to residue 234 in *Photinus pyralis* luciferase and to residue 236 of *Luciola mingrelica*, *Luciola cruciata* or *Luciola lateralis* luciferase (231 in *Phrixothrix),* may also affect the thermostability of the protein. In general, the amino acid at this position is aspartic acid or glycine and in some cases, glutamine or threonine. These are suitably changed for non-polar or different non-polar amino acids as appropriate. For example, in *Photinus pyralis,* the amino acid residue is aspartic acid is suitably replaced with a non polar amino acid, such as alanine, leucine, glycine, valine, isoleucine, proline, methionine or tryptophan. A particularly preferred substitution for the phenylalanine residue corresponding to residue 234 in *Photinus pyralis* luciferase is glycine. Where a non-polar amino acid residue such as glycine is present at this position (for example in *Luciola* luciferase), this may be substituted with a different non-polar amino acid.

Changes of the amino acid residue corresponding to residue 420 in *Photinus pyralis* luciferase and to residue 422 of *Luciola mingrelica, Luciola cruciata* or *Luciola lateralis* luciferase (417 in *Phrixothrix* green and 418 in *Phrixothrix* red), may also affect the thermostability of the protein. In general, the amino acid at this position is an uncharged polar amino acid serine or threonine or glycine. These are suitably changed for different uncharged polar amino acids. For example, in *Photinus pyralis*, the serine may be replaced with asparagine, glutamine, threonine or tyrosine, and in particular threonine.

Changes of the amino acid residue corresponding to residue 310 in *Photinus pyralis* luciferase and to residue 312 of *Luciola mingrelica*, *Luciola cruciata* or *Luciola lateralis* luciferase, may also affect the thermostability of the protein. The amino acid residue at this position varies amongst the known luciferase proteins, being histidine in *Photinus pyralis*, *Pyrocelia miyako, Lampyris noctiluca* and some forms of *Photuris pennsylanvanica* luciferase, threonine in *Luciola mingrelica*, *Hotaria parvula* and *Phrixothix* (where it is amino acid 307) luciferase, valine in *Luciola cruciata* and *Luciola lateralis,* and asparagine in some *Pyrophorus plagiophthalamus* luciferase. Thus, in general, the amino acid at this position is hydrophilic amino acid which may be changed for a different amino acid residue which increases thermostability of the enzyme. A particularly preferred substitution for the histidine residue corresponding to residue 310 in *Photinus pyralis* luciferase is arginine.

Other mutations may also be present in the enzyme. For example, in a preferred embodiment, the protein also has the amino acid at position corresponding to amino acid 354 of the *Photinus pyralis* luciferase (356 in Luciola luciferase) changed from glutamate, in particular to an amino acid other than glycine, proline or aspartic acid. Suitably, the amino acid at this position is tryptophan, valine, leucine, isoleucine are asparagine, but most preferably is lysine or arginine. This mutation is described in WO 95/25798. It has been found that hydrophobic residues at this position enhance the wavelength shift of the enzyme. Furthermore, the presence of a large hydrophobic (V or I), polar (N) or positively charged (K or R) amino acid at position 354 enhances thermostability.

In an alternative preferred embodiment, the protein also has the amino acid at the position corresponding to amino acid 217 in Luciola luciferase (215 in *Photinus pyralis)* changed to a hydrophobic amino acid in particular to isoleucine, leucine or valine as described in EP-A-0524448.

Proteins of the invention are recombinant luciferase enzymes. They may have at least 90% similarity to wild sequences such as those of *Photinus* pyralis, *Luciola mingrelica, Luciola*, *cruciata* or *Luciola lateralis* enzyme in the sense that at least 90% of the amino acids present in the wild-type enzymes are present in the proteins of the invention. Similar proteins are of this type include allelic variants, proteins from other insect species as well as recombinantly produced enzymes. They can be readily identified in that they are encoded by nucleic acids which hybridise with sequences which encode wild-type enzymes under stringent hybridisation conditions. Such conditions would be well understood by the person skilled in the art, and are exemplified for example in Sambrook et al. (1989) Molecular Cloning, Cold Spring Harbor Laboratory Press). In general terms, low stringency conditions can be defined as 3 x SCC at about ambient temperature to about 65°C, and high stringency conditions as 0.1 x SSC at about 65°C. SSC is the name of a buffer of 0.15M NaCl, 0.015M trisodium citrate. 3 x SSC is three times as strong as SSC and so on.

In particular, the similarity of a particular sequence to the sequences of the invention may be assessed using the multiple alignment method described by Lipman and Pearson, (Lipman, D.J. & Pearson, W.R. (1985) Rapid and Sensitive Protein Similarity Searches, Science, vol 227, pp1435-1441). The "optimised" percentage score should be calculated with the following parameters for the Lipman-Pearson algorithm:ktup =1, gap penalty =4 and gap penalty length =12. The sequences for which similarity is to be assessed should be used as the "test sequence" which means that the base sequence for the comparison, such as the sequence of *Photinus pyralis* or any of the other sequences as recorded in Ye et al., supra., should be entered first into the algorithm.

Particular examples of proteins of the invention are wild-type luciferase sequence with one or more of the mutations as outlined above.

The invention further provides nucleic acids which encode the luciferases as described above. Suitably, the nucleic acids are based upon wild-type sequences which are well known in the art. Suitable mutation to effect the desired mutation in the amino acid sequence would be readily apparent, based upon a knowledge of the genetic code.

In a preferred embodiment of the invention, the nucleic acid is a synthetic gene. Suitably, the synthetic gene is engineered to remove codons rarely found in highly expressed genes from common expression hosts such as E. *coli* and, at the same time, avoid the introduction of codons rarely found in genes coding for beetle luciferases. This approach ensures that the new gene has a codon utilisation that is optimal for both E. *coli* and insect expression systems.

For example, wherever possible the codons for the amino acids arg, leu, ile, gly and pro were changed to CGT or CGC (arg), CTG, CTT or CTC (leu), ATC or ATT (ile), GGT or GGC (gly), and CCG CCA or CCT (pro), thus eliminating rare codons. In the case of the synthetic gene illustrated below (SEQ ID NO 1) and in Figure 14, this resulted in a total of 139 silent mutations creating 62 new non-rare codons (11% of the total). The first 8 nucleotides shown in Figure 14 form part of the ribosome binding site and thus do not code. The coding sequence begins with the methionine residue indicated by an up arrow. This coding sequence and closely similar sequences, for example sequences which have at least 90% similarity or preferably at least 95% similarity form a preferred aspect of the invention.

Another useful feature which may be employed when producing a synthetic assembly is the incorporation of new unique restriction sites. These sites make mutagenesis, in particular combinatorial cassette mutagenesis, of the gene simpler and more efficient. In particular, it may be desirable to create unique restriction sites within the cDNA coding for subdomain B in the enzyme. Additionally creation of a unique restriction site at the extreme 3' end of the gene to allow simple fusions and/or removal of the peroxisome targeting sequence may be advantageous.

In the example illustrated hereinafter, nine new unique restriction sites were engineered, mostly in the central third of the gene, and a unique Hind III site was generated at the extreme 3' end of the gene to allow for simple C-terminal fusions (Figure 12).

Finally, use of a synthetic gene allows for the introduction of mutations to increase the thermostablilty of the gene product, or to otherwise modify the properties of the product as desired. In the Example illustrated hereinafter for instance, three non-silent mutations were engineered to introduce the thermostabilising amino acid changes T214C, E354K and D357F into the polypeptide.

The nucleic acids of the invention are suitably incorporated into an expression vector such as a plasmid under the control of control elements such as promoters, enhancers, terminators etc. These vectors can then be used to transform a host cell, for example a prokaryotic or eukaryotic cell such as a plant or animal cell, but in particular a prokaryotic cell such as E. *coli* so that the cell expresses the desired luciferase enzyme. Culture of the thus transformed cells using conditions which are well known in the art will result in the production of the luciferase enzyme which can then be separated from the culture medium. Where the cells are plant or animal cells, plants or animals may be propagated from said cells. The protein may then be extracted from the plants, or in the case of transgenic animals, the proteins may be recovered from milk. Vectors, transformed cells, transgenic plants and animals and methods of producing enzyme by culturing these cells all form further aspects of the invention.

The *Photinus pyralis* D357Y mutant luciferase was created by random mutagenesis as described hereinafter. It was found that the D357Y single point mutation produces a large colour shift in the wavelength of light emitted and also has greater thermostability than wild type luciferase. Further investigations have revealed that a range of substitutions at this position give rise to good thermostability and/or to large colour shifts.

Particular examples of mutant enzymes of *Photinus pyralis* which fall within the scope of the invention include the following:

D357Y
D357F
D357W
D357K
D357N
D357I
E354I/D357Y
E354V/D357Y
E354C/D357Y
E354R/D357Y
E354S/D357Y
E354N/D357Y
E354K/D357M
E354R/D357L
E354W/D357W

E354H/D357W
E354R/D357F
E354K/D357F
E354S/D357F
E354M/D357F
E354A/D357R
E354A/D357F
E354T/D357Y
E354A/D357N
I351M/E354R/D357V
E354S/D357V
E354R/D357W
E354R/D357M
E354R/D357S
E354N/D357S

or equivalents of any of these when derived from the luciferases of other species.

The mutations for the creation of the above mutants were introduced to the luciferase gene on plasmid pET23 by site-directed mutagenesis, (PCR) or combinatorial cassette mutagenesis. The oligonucleotides added to the PCR reaction in order to effect the relevant mutations are given below.

It has been reported previously that the effect of point mutations at the 354 and 215 positions are additive. This invention provides the possibility of combining three or more such mutations to provide high thermostability in a mutant enzyme which has a large colour shift.

Luciferase proteins of the invention will advantageously be employed in any bioluminescent assay which utilises the luciferase/luciferin reaction as a signalling means. There are many such assays known in the literature. The proteins may therefore be included in kits prepared with a view to performing such assays, optionally with luciferin and any other reagents required to perform the particular assay.

The invention will now be particularly described by way of example with reference to the accompanying diagrammatic drawings in which:

Figure 1 is a log graph showing % remaining activity versus time of 45°C incubation of several mutant enzymes in accordance with the invention;

Figure 2 shows the spectral peaks obtained by incubating *E. coli* cells expresing luciferase enzymes in a citrate buffer with D-luciferin where where the enzyme used is (a) recombinant wild-type *Photinus pyralis* luciferase, (b) a D357K mutant, (c) a D357N mutant, (d) a D357W mutant, (e) a D357I mutant, (f) a D357F mutant, (g) a D357Y mutant and (h) a double mutant E354I + D357Y;

Figure 3 is a graph showing the % remaining activity versus time of three mutant enzymes, E354I, D357Y and the double mutant (DM) E354I/D357Y;

Figure 4 shows the emission spectra of (a) recombinant wild type enzyme and (b) the double mutant (DM) E354I/D357Y;

Figure 5 is a graph showing the rate decay of photon emissions of recombinant wild-type (♦) r-wt and a D357K mutant enzyme (|).

Figure 6 shows molecular modelling diagram, illustrating a potential CoA binding pocket within the luciferase enzyme;

Figure 7 shows the in vivo bioluminescent spectra emitted by E. coli cells expressing mutant *P. pyralis* luciferase D357Y (a) growth on LB; (b) growth on minimal medium and sodium acetate; (c) growth on minimal medium and glucose;

Figure 8 shows the in vivo bioluminescent spectra emitted by E. coli cells expressing mutant *P. pyralis* luciferase E354K/D357M (a) growth on LB; (b) growth on minimal medium and sodium acetate; (c) growth on minimal medium and glucose;

Figure 9 is a graph showing the effect of CoA on spectral distribution of light emitted by mutant *P. pyralis* luciferase D357Y;

Figure 10 is a graph showing the normalised data of the effect of CoA on spectral distribution of light emitted by mutant *P. pyralis* luciferase D357Y;

Figure 11 is a graph showing the effect of CoA on spectral distribution of light emitted by mutant *P. pyralis* luciferase E354I/D357Y (Figure 11a) and normalised data (Figure 11b);

Figure 12 illustrates the restriction sites modifications utilised in the construction of a synthetic luciferase gene;

Figure 13 illustrates constructs used in the synthesis of a luciferase gene;

Figure 14 shows the cDNA sequence (SEQ ID NO 1) of the synthetic luciferase gene (including nucleotides 1-8 which form part of the ribosome binding site but are not coding) and the encoded amino acid sequence which starts at the methionine residue indicated by the up arrow (SEQ ID NO 2); and

Figure 15 illustrates the thermostability of mutants including the mutant encoded by the synthetic gene at 50°C.

Example 1

Identification and Characterisation of Mutant Luciferase

Two libraries of firefly (*Photinus pyralis)* luciferase, created used error-prone PCR [M. Fromant et al., Anal. Biochem. (1995) 224, 347-353], were prepared. One library comprised of error-prone PCR products of the full length *luc* gene, cloned into the T7 expression system pET23a, (Novagen Inc., Madison, WI, U.S.A.). A second library consisted of the error-prone PCR products of a short section of the *luc* gene, covering amino acids 199-352, cloned in the vector pBSK(+), (Stratagene, La Jolla, CA, U.S.A.).

The pET23a library was expressed in *E. coli* strain BL21(DE3), (*E. coli* B F *dcm ompT hsdS(*$r_B^-m_B^-$*) gal*λ (DE3)).

The pBSK(+) library was expressed in HB101 *E. coli* cells, (*supE44 aral4 galK2 lacY1* Δ(*gpt-proA*) *62 rpsL20 (Str*$^r$*) xyl-5 mtl-1 recA13* Δ (*mrcC-mrr*) *HsdS*$^-$ (*r*$^-$*m*$^-$). pET23a and pBSK (+) both carry the gene for β-lactamase and confer ampicillin resistance to E. *coli* cells harbouring the plasmid.

An E. *coli* strain was transformed with the prepared library by electroporation, using a BIORAD E. *coli* Pulser, and grown overnight at 37°C on LB agar, containing ampicillin at a concentration of 50μg/ml. The cells were transferred to nylon membranes, (Osmonics, Minnetonka, Minnesota, U.S.A.), and sprayed with luciferin solution (500μM D-luciferin, potassium salt, in 100mM sodium citrate buffer, pH 5.0). The colonies were viewed using an AlphaImager™ 1200 Documentation and Analysis System (Flowgen, Lichfield, Staffordshire, UK). This integrated the bioluminescence emitted over a specified period of time to produce an image of the light emitted by the colonies. The brightness of luminescence was taken as an indication of the thermostability of luciferase.

The colonies were then screened for thermostability. Colonies were selected on the basis of brightness of light emitted and were isolated for further characterisation. In some screens, the E. *coli* colonies were incubated at 42°C for 2 hours prior to screening so that the thermostable mutants could be selected. Colonies isolated from the primary screen were patched onto nylon membranes and also grown overnight in LB medium containing ampicillin. The patches were sprayed with luciferin solution and viewed in the AlphaImager™. This secondary screen helped to positively identify clones for in vitro analysis of luciferase activity. *E. coli* clones expressing possible thermostable enzymes were assayed in vitro for luciferase activity and thermostability.

In vitro assays for luciferase activity were performed at room temperature using the Promega Luciferase Assay System (Promega Corporation, Madison, WI, U.S.A.).

The luciferase reaction was initiated by the addition of 10μl crude cell extract to 100μl Promega Luciferase Assay Cocktail (1 in 2 dilution). The resultant bioluminescence was measured using a Biotrace M3 luminometer.

Crude cell extracts were prepared as described in the Promega technical bulletin no. 101. Aliquots of E. *coli* overnight cultures were lysed in cell culture lysis reagent, (25mM Tris-phosphate pH7.8, 2mM dithiothreitol (DTT), 2mM 1,2-diaminocyclohexame-N,N,N',N'-tetraacetic acid, 10% glycerol, 1% Triton X-100, 1.25 mg/ml hen lysozyme) for 10 minutes at room temperature. Crude lysate were then stored on ice prior to assay.

The properties of the enzymes were further tested in time-dependent inactivation studies. Eppendorf tubes containing 50μl aliquots of crude cell extract were incubated in a water bath at a given temperature. At set time points tubes were removed and cooled on ice prior to assay. Remaining luciferase activity was expressed as a percentage of the original activity.

Log graphs of percentage remaining activity versus time of incubation were plotted and used to calculate $t_{1/2}$

values. $T_{1/2}$ is the time taken for the enzyme to lose 50% of its original activity after incubation at a given temperature. $T_{1/2}$ values, (time for activity to reduce to 50% of original activity), were determined in crude extracts at 37°C from log graphs of % remaining activity versus time (not shown).

Plasmid DNA from E. *coli* clones expressing the most thermostable luciferase as determined above, was sequenced in order to determine the mutations responsible for the thermostability of the enzyme.

Plasmid DNA was prepared using the QIAGEN QIAprep Spin Miniprep Kit, (QIAGEN Ltd, Crawley, W. Sussex, UK), following the protocol for using a microcentrifuge (QIAprep Miniprep Handbook 04/98).

All DNA sequencing was undertaken by Babraham Tech$^{nix}$, Cambridge, UK, using an ABI PRISM™ 377 DNA Sequencer and the ABI PRISM™ BigDye™ Terminator Cycle Sequencing Ready Reaction Kit (Perkin Elmer Applied Biosystems) which is based upon the dideoxy chain termination method [F. Sanger et al., Proc. Natl. Acad. Sci. U.S.A. 74, (1977) 5463-5467].

As a result of this work, the novel mutant D357Y was identified.

The crystal structure of luciferase [E. Conti et al., Structure, 4 (1996) 287-298] shows that position 357 is situated on the surface of the protein and is close to position 354, which can affect both thermostability and spectral properties. This indicates that this region could be important in terms of the thermostability of the enzyme.

D357Y is a particularly thermostable mutant, being the most thermostable luciferase, with a single amino acid change.

## Example 2

### Site-Directed Mutagenesis to create other 357 mutants

In order to evaluate different mutations at the 357 position, site-directed mutagenesis was performed using the Stratagene QuikChange™ Site-Directed Mutagenesis Kit, (Stratagene, La Jolla, CA, U.S.A.). The plasmid pPW601a J54, (PJW, MoD Report, 3/96), was used in all site-directed mutagenesis. All products of the mutagenesis reactions were transformed into *E. coli* strain XL1-Blue, [*e14*$^-$ (*mcrA*$^-$) Δ (*mcrCB-hsdSMR-mrr*) *171 endA1 supE44 thi-1 gyrA96 relAl lac recB recJ sbcC umuC*::*Tn5* (*Kan*$^r$) *uvrC [F' proAB lacI*$^q$*Z*Δ*M15 Tn 10* (*Tet*$^r$) *Amy Cam*$^r$]] Oligonucleotide primers were synthesized by Sigma-Genosys Ltd., Cambridge, UK and were designed using an intelligent doping system [A.R Arkin et al., Bio-technology, (1992)10, 297-300, W,. Huang et al., Anal. Biochem. 218, 454-457] were used to design degenerate oligonucleotide primers to produce groups of possible mutations rather than using individual primers for each amino acid substitution.

In this way, libraries of amino acid substituted luciferase mutants were produced.

The following oligonucleotides (and their complementary partners) were used:

| Oligonucleotide Primer (5'->3') | Amino Acid Substitution |
|---|---|
| cacccgagggggat[tat]aaaccgggcgcgg (SEQ ID NO 4) | Y |
| cacccgagggggat[(gac)(tc)(c)]aaaccgggcgcggtcgg (SEQ ID NO 5) | A,I,L,T,V,P |
| cacccgagggggat[(t)(gat)(gc)]aaaccgggcgcggtcgg (SEQ ID NO 6) | C,F,L,W,Y,X |
| cacccgagggggat[(ac)(ga)(gc)]aaaccgggcgcggtcgg (SEQ ID NO 7) | R,S,K,N,H,Q |

The libraries of mutants were screened as previously for thermostability. The number of colonies to be screened was calculated using the equation [S. Climie et al., J. Biol. Chem. 265 (1990) 18776-18779]

$$N = [\ln(1-P)]/[\ln((n-1)/n)]$$

Where N is the number of colonies to be screened, n is the number of possible codons at the target position and P is the probability that every codon in the mixture is sampled for screening at least once. The calculation was based on P=0.95. The mutants obtained from site-directed mutagenesis were assayed for luciferase activity and characterised in time-dependent thermoinactivation studies.

Mutants identified as desirable in this way were grown in 400ml LB medium, containing ampicillin, to $A_{260} \approx 0.5$. Luciferase expression was then induced by addition of isopropyl β-thiogalactoside (IPTG) to a final concentration of 1mM. The cells were then incubated at 30°C, with shaking, for 3 hours prior to harvesting by centrifugation. The resultant cell pellet was resuspended in 10ml B-PER™ Protein Extraction Reagent, (Pierce Chemical Company, Rochford, U.S.A.), 1mM DTT to produce a crude extract, following the B-PER™ protocol for Maxi-Scale Bacterial Protein Extraction.

Reconstituted Sigma Protease Inhibitor Cocktail, 500μl, (Product No. P8465, Sigma, Saint Louis, Missouri, U.S.A.), was added to the B-PER™ solution to inhibit endogenous proteases. The cell lysate was then centrifuged at 30 OOOg for 30 minutes.

The supernatant of the crude extract was subjected to fractionation with ammonium sulphate. The fraction that precipitated between 30% and 55% saturation contained luciferase activity. This material was resuspended in 0.5ml Tris HC1 pH8.0, 1mM DTT and used for thermoinactivation and spectral studies.

The replacements D357L, T, V, W, R, I, S, K, N and F were introduced. These mutants were characterised in *in vitro* thermoinactivation studies of crude extracts.

The partially purified extracts were diluted, 1 in 11, into a thermoinactivation buffer: 50mM potassium phosphate buffer pH7.8 containing 10% saturated ammonium sulphate, 1mM dithiothreitol and 0.2% BSA.

110μl aliquots of protein solution were incubated at 40°C or 45°C for set periods of time and cooled on ice prior to assay. Luciferase activity was then measured as described in Example 1, using Promega Luciferase Assay Reagent (1 in 2 dilution).

The results are shown in Tables 2 & 3 and in Figure 1. T1/2 values were determined in crude extracts at 40°C (Table 2) and 45°C (Table 3).

Table 2

| Mutant | $T_{1/2}$ |
|---|---|
| D357K | 2.2 |
| D357R | 4.2 |
| D357S | 4.6 |
| D357N | 4.8 |
| D357V | 5.9 |
| D357T | 7.3 |
| D357L | 11.3 |
| D357I | 18.0 |
| rWT | <1.0 |

Table 3

| Mutant | $T_{1/2}$ |
|---|---|
| D357W | 2.5 |
| D357F | 6.5 |
| D357Y | 10.4 |
| RWT | <1.0 |

All the substitutions displayed enhanced thermostability in comparison to recombinant wild type.

Example 3

Changes in Wavelength of Emitted Light

Amino acid replacements at position 357 were also observed to affect the *in vivo* spectra of light emitted by the enzyme. An aliquot, (250μl), of *E. coli* cell cultures, as described in Example 2 were grown overnight at 37°C, was spun down in a microcentrifuge and the supernatant removed. Cells expressing different mutant luciferases were incubated in a citrate buffer (pH5.0) containing 150μl D-luciferin and the light emitted from the *in vivo* reaction was analysed by measuring the emission spectra using a SPECTRAmax® Microplate Spectrofluorometer, (Molecular Devices Corp. California, U.S.A.). Large changes in the spectral peak as well as the distribution of wavelengths was observed for the mutants D357Y, F and I (Figure 2(a)-(g)). These results are summarised in Table 4 below.

In addition, the *in vivo* luminescence of the mutants was assessed by eye in a dark room. The D357 mutants displayed a variety of colours in their luminescence spectra. In particular, D357Y, F and I showed significant shifts to longer wavelengths of emitted light.

In some cases, (e.g. D357F), the change in colour of light emission appeared to be due, not only to a shift in $\lambda_{max}$, but to a difference in contributions to the spectra from different wavelengths of visible light.

Table 4

| Mutant | $\lambda_{max}$ (nm) | Deviation from rWT (nm) |
|---|---|---|
| rWT | 558 | - |
| D357K | 556 | -2 |
| D357N | 558 | 0 |
| D357W | 558 | 0 |
| D357I | 606 | +48 |
| D357F | 611 | +53 |
| D357Y | 613 | +55 |

Recombinant wild type (r-wt) enzyme was used for comparison of $\lambda_{max}$ of *in vivo* light emission of some of the 357 mutants. D357Y, F and I display considerable shifts in their wavelength maxima.

Example 4

Enzyme properties in the presence or absence of CoA

D357Y was partially purified by ammonium sulphate precipitation, as described in the Example 1. This partially purified D357Y enzyme (5μl) was mixed with 150μl Promega Luciferase Assay Reagent. Another aliquot was mixed with an equivalent assay buffer in which CoA is absent, (25mM Tris Tricine pH7.8, 5.0mM $MgSO_4$, 0.1mM EDTA, 2mM DTT, 470μM D-luciferin, 530μM ATP). The emission spectra of the two reactions were measured and are shown in Figures 9 and 10.

The spectra display a marked difference in bioluminescent emission in the absence and presence of CoA, with dramatic shift in $\lambda_{max}$. The effect of CoA on the kinetics of the luciferase reaction can also be seen by in the difference in RLU scales. (RLU - Relative Light Units).

This difference in emission gives rise to the possibility of using the enzyme in an assay to detect the presence of CoA.

Example 5

Preparation and Properties of Double Mutant

Using site-directed mutagensis as described in Example 2, a double mutant of E354I + D357Y was engineered in order to study any cumulative effects upon thermostability and colour of light emission.

The partially purified double mutant, E354I + D357Y, was diluted, 1 in 11, into a thermoinactivation buffer: 50mM potassium phosphate buffer pH7.8 containing 10% saturated ammonium sulphate, 1mM dithiothreitol and 0.2% BSA.

110μl aliquots of protein solution were incubated at 45°C for set periods of time and cooled on ice prior to assay. Luciferase activity was then measured as previously, using Promega Luciferase Assay Reagent (1 in 2 dilution).

The double mutant displayed a marked increase in thermostability in comparison to the single mutants E354I and D357Y individually, (see Figure 3). Thermoinactivation studies of partially purified double mutant confirmed the increased thermostability of the mutant, giving a $t_{1/2}$ value of 7.7 min when inactivated at 45°C.

It was noted that the double mutant displays a much deeper red colour of luminescence than the individual mutants of E354I and D357Y, displaying additivity of colour of luminescence.

The emission spectra of recombinant wild type and the crude extract of the double mutant E354I + D357Y were also measured using the assay buffer described in Example 3.

Emission spectra measured *in vivo* give a $\lambda_{max}$ of 611nm. However, the spectrum has a greater contribution of luminescence from the red region of wavelengths, leading to its deeper red appearance when visualised by eye. Emission spectra in crude extracts displayed a definite change in spectral shape and a wavelength shift of 44nm, relative to rWT, (see Figure 4).

The *in vivo* emission spectrum of the double mutant shows both a sharpening of the bandwidth for the peak wavelength of emitted light (613nm) and a decrease in the contribution from wavelengths of light in the region 540-560nm.

The dramatic effect of these mutations indicates the importance of this region of the enzyme to the colour of bioluminescent light.

Example 6

Improved Photon Flux

The *in vivo* bioluminescence of *E. coli* cells expressing the mutant D357K was observed to be very bright relative to the other mutants at this position. The flash kinetics of this enzyme was analysed using a luminometer, which could measure the rate of photon emission over time. Aliquots of E. *coli* cell free extracts containing recombinant wild type enzyme or the mutant D357 were added to a luciferase assay cocktail, which did not contain any reagents that would promote glow kinetics e.g. coenzymeA. The rate of decay of photon emission was measured over time (15s) for both enzymes was observed to be significantly slower for the mutant D357K (Figure 4). In other words the mutant enzyme has reaction kinetics, which are inhibited to a lesser degree, over at least the first 15 seconds of the reaction, than the recombinant wild type enzyme.

Example 7

Combinatorial cassette mutagenesis at positions E354 and D357 Step 1

Engineering plasmid pPW601aJ54 for cassette mutagenesis

Two new unique restriction sites were introduced into the *luc* gene, in the plasmid pPW601a/J54, using two pairs of synthetic oligonucleotides (see below). A total of six silent mutations introduced a SpeI and a KpnI restriction site, 63 base pairs apart, within the gene. Plasmid containing these new sites was called pPW601aJ54SpeI/KpnI. The presence and proximity of these restriction sites makes it possible to use combinatorial cassette mutagenesis to explore the effects of random substitutions at amino acid positions 354 and 357 in the primary sequence of firefly luciferase.

SpeI (a) 5'-gggctcactgagact**acTAGT**gctattatgattacacccg-3' nt1021- nt1060 (SEQ ID NO 8)

SpeI (b) 5'-cgggtgtaatcagaatagc**ACTAgt**agtctcagtgagccc-3' (SEQ ID NO 9)

KpnI (a) 5'-ggcgcggtcggtaaagt**GgtAcc**attttttgaagcg-3' nt1078-nt1113 (SEQ ID NO 10)

KpnI (b) 5'-cgcttcaaaaaat**ggTacCa**ctttaccgaccgcgcc-3' (SEQ ID NO 11)

Nucleotides highlighted in bold form the endonuclease recognition site and those in upper case the position of the point mutations necessary to create the site.

Step 2

Cassette design and library construction

A pair of synthetic oligonucleotides was synthesised which when annealed created a double stranded cassette which could be ligated directly into plasmid pPW601aJ54SpeI/KpnI digested at the new restriction sites. The cassette was designed to introduce all possible combinations of the 20 naturally occurring amino acids at positions 354 and 357 in the primary sequence.

Looplib2A 5'-ctagtgctattctgattacacccNNG/CggggatNNG/Caaaccgggcgcggtcggtaaagtg gta-3' (SEQ ID NO 12)

Looplib2B 5'-cactttaccgaccgcgcccggtttG/CNNatccccG/CNNgggtgtaatcagaatagca-3' (SEQ ID NO 13)

2μg of each of the loop library oligonucleotides was mixed in a buffer containing 50mM Tris-HCl pH 7.4, 25mM NaCl, and heated to 100°C for 3min. This solution was then cooled slowly in a heating block to <50°C to anneal the complimentary sequences. The annealed oligonucleotides were then ligated into plasmid pPW601aJ54SpeI/KpnI, which had been digested with SpeI and KpnI. Aliquots of the ligation reaction were then used to transform *E. coli* HB101 cells using electroporation. After electroporation transformed cells were plated out on LB agar plates containing 50μg/ml ampicillin and grown overnight at 37°C. The following day 869 colonies were picked at random from the plates and used to inoculate 1ml of LB containing ampicillin in 96 square-well plates (Beckman). The plates were covered and the cells grown overnight at 37°C with shaking.

Step 3

*In vivo* screening the randomly selected clones

The next morning 50μl aliquots of the stationary phase overnight cultures were transferred to two clear plastic round bottom 96 well microtitre plates (Dynex). One plate was covered and incubated on a heated block for 8 minutes (block surface temperature 45°C) whilst the other was kept at 37°C. The *in vivo* luciferase activity in the cells from both plates was then detected and recorded, at room temperature, by adding 50μl of a 100mM sodium citrate buffer pH 5.0 containing 0.5mM D-luciferin to the wells and then transferring the plate to a video camera imager capture system (Alpha Imager). The light emitted by the heated and control cultures was integrated over 1 or 2 minutes and the image recorded on thermal paper film.

Seventy-nine cultures exhibiting the greatest bioluminescence, as determined by the brightness of the image recorded on film, were selected for a second round of screening. This time the cultures were incubated for 16 minutes on the heating block prior to being assayed. Of the 55 clones selected from the *in vivo* thermostability screens 25 were chosen for *in vivo* spectral analysis. These clones were grown overnight in LB at 37°C and the next morning 200ul of the overnight cultures was centrifuged and the E. *coli* cell pellets were resuspended in 150μl of 100mM sodium citrate buffer pH 5.0 containing 0.5mM D-luciferin. The resuspended cells were then placed in a white plastic microtitre plate and the *in vivo* bioluminescent emission spectrum emitted by each of the mutant luciferases was analysed using a Molecular Devices Spectramax 96 well plate fluorimeter. The results are summarised in the Table 1 below.

Step 4

Identification of mutations

Plasmid DNA was prepared from the 25 clones selected by *in vivo* screening and sequenced using gene specific sequencing primers. Mutations resulting in amino acid changes at positions 354 and 357 in the primary sequence were identified. One mutant, also contained an additional mutation resulting in an amino acid substitution at position I351 (Table 5).

Table 5

| Mutant Enzyme | Mutations | Peak Wavelengths (nm) |
|---|---|---|
| 1 | E354V/D357Y | 614 |
| 2 | E354I/D357Y | 612 |
| 3 | E354C/D357Y | 612 |
| 4 | E354R/D357Y | 600 |
| 5 | E354S/D357Y | 612 |
| 6 | E354N/D357Y | 608 |
| 7 | E354K/D357M | 556,606 |
| 8 | E354R/D357L | 588 |
| 9 | E354W/D357W | 610 |
| 10 | E354H/D357W | 606 |
| 11 | E354R/D357F | 596 |
| 12 | E354K/D357F | 608 |
| 13 | E354S/D357F | 610 |
| 14 | E354M/D357F | 610 |
| 15 | E354A/D357R | 556 |
| 16 | E354A/D357F | 610 |
| 17 | E354T/D357Y | 612 |
| 18 | E354A/D357N | 560 |
| 19 | I351M/E354R/D357V | 606 |
| 20 | E354S/D357V | 556,608 |
| 21 | E354R/D357W | 600 |
| 22 | E354R/D357M | 596 |
| 23 | E354R/D357S | 592 |

(continued)

| Mutant Enzyme | Mutations | Peak Wavelengths (nm) |
|---|---|---|
| 24 | E354N/D357S | 600 |
| rWT | E354/D357 | 552 |

where rWT signifies recombinant wild-type.

A number of mutant luciferases were selected from the *in vivo* assays for thermostability. The majority of these luciferases also show large changes in the *in vivo* spectrum of emitted light with many showing greater contributions from longer wavelengths of light (>580nm). A number of spectra also showed a significant narrowing of bandwidth around a single peak of 610-614nm.

Replacements of E354 and D357 with a hydrophobic and an aromatic amino acid respectively e.g. E354V, D357Y results in the largest change in the in vivo spectrum which shows a single peak, of narrow bandwidth, around 612nm.

Example 8

*In vitro* screening for thermostability

Cell free extracts of the selected clones were prepared by lysis and the thermostability of the luciferase from each extract was determined in a thermal inactivation experiment. 50μl of each extract was placed in an eppendorf tube and incubated in a waterbath heated to 45°C for 4, 9 and 16 minutes. At the appropriate timepoint the aliquot was removed and the remaining luciferase activity measured. Table 6 shows the percent remaining activity versus time for all mutant enzymes as well as recombinant wild type.

Table 6

| Enzyme No. (see Table 5) | Percentage activity remaining after incubation at 45°C | | | |
|---|---|---|---|---|
| | 0 min | 4 min | 9 min | 16 min |
| 1 | 100 | 95 | 87 | 75.4 |
| 2 | 100 | 99 | 84.7 | 67.7 |
| 3 | 100 | 92 | 73 | 53.3 |
| 4 | 100 | 94 | 89 | 71.4 |
| 5 | 100 | 85 | 72.2 | 53 |
| 6 | 100 | 93 | 84.8 | 71 |
| 7 | 100 | 63.7 | 31 | 11.7 |
| 8 | 100 | 58.6 | 19 | 4.9 |
| 9 | 100 | 85.4 | 65.3 | 42.3 |
| 10 | 100 | 65.5 | 27.8 | 10.6 |
| 11 | 100 | 88.6 | 70 | 54 |
| 12 | 100 | 90 | 69 | 52 |
| 13 | 100 | 83 | 60.5 | 39 |
| 14 | 100 | 80 | 61 | 39 |
| 15 | 100 | 1.7 | 0.1 | nd |
| 16 | 100 | 90 | 76 | 63 |
| 17 | 100 | 91 | 78 | 60 |
| 18 | 100 | 19 | 1.8 | nd |
| 19 | 100 | 17 | 1.4 | nd |
| 20 | 100 | 17 | 1.1 | nd |

(continued)

| Enzyme No. (see Table 5) | Percentage activity remaining after incubation at 45°C 0 min | 4 min | 9 min | 16 min |
|---|---|---|---|---|
| 21 | 100 | 71 | 63 | 34 |
| 22 | 100 | 80 | 40 | 21 |
| 23 | 100 | 29 | 4 | 0.6 |
| 24 | 100 | 28 | 4 | 0.4 |
| 25 (D357K) | 100 | 0.1 | nd | nd |
| rWT | 100 | 0.05 | nd | nd |
| where "nd" indicates not done. | | | | |

The results indicate that the most thermostable luciferases were those with an aromatic amino acid at position 357 (Y, F or W) and a large hydrophobic (V or I), polar (N) or positively charged (K or R) amino acid at position 354.

Example 9

Effect of growth conditions on the in vivo spectrum of emitted light.

The effect of different carbon sources on the spectrum of emitted light from E. *coli* BL21(DE3) cells expressing mutant luciferases D357Y or E354K + D357M (7 above) was investigated.

A 50ml culture of cells was grown to mid log phase on LB medium and then harvested by centrifugation. The cell pellet was resuspended in 1ml of sterile distilled water and a 100ul aliquot of this suspension was then used to inoculate 5ml of fresh LB, M9 minimal medium + 2mM sodium acetate or M9 minimal medium + 2mM glucose in a 25ml Sterilin tube. The cultures were allowed to continue growing, at 37°C with shaking, and after 90 minutes (D357Y) or 120 minutes (enzyme 7) a 200μl aliquot of cells was removed centrifuged and resuspended in 150ul of 100mM sodium citrate buffer pH 5.0 containing 0.5mM D-luciferin. The resuspended cells were then placed in a microtitre plate and the *in vivo* bioluminescent emission spectrum emitted by each of the mutant luciferases was analysed using a Molecular Devices Spectramax 96 well plate fluorimeter. The results are shown in Figures 7 and 8.

The results show that switching from a rich medium (LB) (Figure 7a, 8a) to a defined minimal medium with either acetate (Figure 7b, 8b) or glucose (Figure 7c, 8c) as the sole carbon source resulted in shifts to longer wavelengths of emitted light and a reduction in the contribution from shorter wavelengths.

Example 10

Purification and spectral characterisation of recombinant wild type, and mutant luciferases

Recombinant wild type *Photinus pyralis* enzyme and the mutant luciferases D357Y and E354I + D357Y were purified to homogeneity in order to analyse the effect of the cofactor coenzyme A on the spectrum of the bioluminescent reaction. All three luciferases were purified as fusions to a 143 amino acid carbohydrate binding module (CBM) from the anaerobic fungus *Piromyces equii.* This CBM has been shown to bind selectively to acid swollen cellulose and the soluble carbohydrates galactomannan and glucomannan, forming the basis for a simple single step affinity purification scheme.

Luciferases fused to the CBM can be bound to cellulose in crude cell free extracts, washed, and then eluted selectively using soluble polysaccharides. Fusion proteins purified this way were used in assays to measure the wavelengths of emitted light in reactions containing different amounts of coenzyme A. Enzyme (5μl) was added to 100μl of assay reagent, 25mM Tris-Tricine pH 7.8, 5.0mM $MgSO_4$, 0.1mM EDTA, 530μM ATP and 470μM D-luciferin, containing different amounts of coenzyme A. Figures 9-11 show the effect of increasing concentrations of coenzyme A on the spectrum of light emitted by the purified luciferases D357 and E354I + D357Y.

*In vivo* assays of the spectrum of bioluminescent light emitted by *E. coli* cells expressing firefly luciferase fused to the C-terminus of the fungal CBM did not show any significant differences from cells expressing the native luciferase Similarly, *in vitro* assays of the spectrum of bioluminescent light emitted by a commercial source of purified recombinant luciferase (Promega) were identical to the spectrum emitted by the fusion protein.

The observed differences are therefore associated with the concentrations of CoA. As the concentration of coenzyme A increases the spectral distribution alters and at the highest concentrations of CoA the spectrum is dominated by wavelengths in the region 590-630nm with a pronounced peak at 610nm. The spectral shift is most marked for the double mutant where there is a significant narrowing of bandwidth around a single peak of wavelength 610nm (Figure 11).

Example 12

Production of synthetic Photinus pyralis luciferase mutated such that it has 214C/354K/357F

A synthetic luc gene was designed and assembled from oligonucleotide pairs using the synthesis strategy outlined above. The gene sequence was engineered to create a luciferase with the amino acids 214C, 354K and 357F.

Twenty-nine pairs of overlapping synthetic oligonucleotides were synthesised by Sigma-Genosys Ltd, purified by PAGE and ligated in three assemblies of approximately 550bp (IDRIS 1,2 & 3, Figure 13). Each assembly was then ligated separately into the vector pBSK(+) and the resulting constructs were used to transform *E. coli* XL1-Blue cells. Plasmid DNA was prepared from clones containing the assembled inserts and sequenced to confirm the fidelity of the ORFs. The presence of n-1 oligonucleotides (by-products of the oligosynthesis) in the assemblies complicated the build process. DNA sequencing identified a single correct assembly of IDRIS 2 and the PCR was used to correct one assembly of IDRIS 3 which contained a single base pair deletion at the 5' end of the construct. Assembly of the complete ORF was achieved by ligating a mixture of plasmids containing IDRIS 1 with IDRIS 2 and 3.

The ligated DNA was then used to transform *E. coli* XL1-Blue cells and clones expressing active enzyme were selected using an *in vivo* assay. Several clones were selected and sequenced to confirm the presence and fidelity of the synthetic *luc* gene having the sequence shown in Figure 14. The complete ORF was called IDRIS (FA).

The synthetic gene was assembled into the vector pBSK(+) between the BamH I and Sal I sites in the polylinker. In this position the gene is not in frame with the alpha peptide and is a significant distance from the lac promoter. However, enough luciferase is produced to enable preliminary characterisation of the enzyme. Crude cell free extracts of E. *coli* XL1-Blue cells expressing IDRIS (FA) were prepared, from overnight cultures, using the Promega lysis method.

The thermostability of the enzyme in the extract was then tested at 50°C over 20 minutes and compared with the thermostable mutant E354I+D357Y. The new codon optimised triple mutant was significantly more thermostable than the mutant E354I+D357Y (Figure 15).

**Claims**

1. A recombinant protein having luciferase activity and at least 90% similarity to a wild-type luciferase from *Photinus pyralis, Luciola mingrelica, Luciola cruciata* or *Luciola lateralis*, *Hotaria parvula, Pyrophorus plagiophthalamus, Lampyris noctiluca, Pyrocoelia miyako,* or *Phrixothrix* wherein in the sequence of the enzyme, the amino acid residue corresponding to residue 357 in *Photinus pyralis* luciferase is mutated as compared to the corresponding wild-type luciferase, such that the luciferase enzyme is able to emit light at a different wavelength as compared to the corresponding wild-type luciferase and/or has enhanced thermostability as compared to the corresponding wild-type luciferase, and wherein similarity is determined using an alignment algorithm as defined by Lipman and Pearson (1985, Science 227: 1435-1441) with the parameters ktup = 1, gap penalty = 4 and gap penalty length =12.

**2.** A recombinant protein according to Claim 1 wherein the wild type luciferase sequence is of luciferase from *Photinus pyralis, Luciola mingrelica, Luciola cruciata or Luciola lateralis.*

**3.** A recombinant protein according to claim 1 wherein said wild-type luciferase sequence is of luciferase from *Photinus pyralis, Luciola mingrelica, Luciola cruciata* or *Luciola lateralis, Hotaria parvula, Pyrophorus plagiophthalamus, Lampyris noctiluca* or *Pyrocoelia miyako* and the amino acid residue corresponding to residue 357 in *Photinus pyralis* luciferase is mutated to other than aspartic acid or glutamic acid.

**4.** A recombinant protein according to any one of the preceding claims wherein the amino acid residue corresponding to residue 357 in *Photinus pyralis* luciferase is mutated to other than aspartic acid, glutamic acid or valine.

**5.** A recombinant protein according to claim 1 wherein said wild-type luciferase sequence is of luciferase from *Phrixothrix* and the amino acid residue corresponding to residue 357 in *Photinus pyralis* luciferase is mutated to other than leucine.

**6.** A recombinant protein according any one of the preceding claims wherein the amino acid residue corresponding to residue 357 in *Photinus pyralis* luciferase is mutated to tyrosine, phenylalanine, tryptophan, asparagines, glutamine, serine or threonine.

**7.** A recombinant protein according to claim 6 wherein the amino acid residue corresponding to residue 357 in *Photinus pyralis* luciferase is mutated to tyrosine.

**8.** A protein according to any one of the preceding claims which has at least one of the following mutations as compared to wild type luciferase:

(a) the amino acid residue corresponding to amino acid 354 of the *Photinus pyralis* luciferase or to amino acid 356 in *Luciola* luciferase is mutated;
(b) the amino acid residue corresponding to position 215 in *Photinus pyralis* luciferase or to position 217 in *Luciola* luciferase) is a different hydrophobic amino acid; or
(c) the amino acid residue corresponding to residue 214 in *Photinus pyralis* luciferase or to residue 216 of *Luciola mingrelica, Luciola cruciata* or *Luciola lateralis* luciferase;
(d) the amino acid residue corresponding to residue 232 in *Photinus pyralis* luciferase or to residue 234 of *Luciola mingrelica*, *Luciola cruciata* or *Luciola lateralis* luciferase;
(e) amino acid residue corresponding to residue 295 in *Photinus pyralis* luciferase or to residue 297 of *Luciola mingrelica, Luciola cruciata* or *Luciola lateralis* luciferase;
(f) amino acid residue corresponding to amino acid 14 of the *Photinus pyralis* luciferase or to residue 16 of *Luciola mingrelica*, or 17 in *Luciola cruciata* or *Luciola lateralis* luciferase;
(g) amino acid residue corresponding to amino acid 35 of the *Photinus pyralis* luciferase or to residue 37 of *Luciola mingrelica,* or to residue 38 of *Luciola cruciata* or *Luciola lateralis* luciferase;
(h) amino acid residue corresponding to amino acid residue 105 of the *Photinus pyralis* luciferase or to residue 106 of *Luciola mingrelica*, 107 of *Luciola cruciata* or *Luciola lateralis* luciferase;
(i) amino acid residue corresponding to amino acid residue 234 of the *Photinus pyralis* luciferase or to residue 236 of *Luciola mingrelica, Luciola cruciata* or *Luciola lateralis* luciferase;
(j) amino acid residue corresponding to amino acid residue 420 of the *Photinus pyralis* luciferase or to residue 422 of *Luciola mingrelica, Luciola cruciata* or *Luciola lateralis* luciferase;
(k) amino acid residue corresponding to amino acid residue 310 of the *Photinus pyralis* luciferase or to residue 312 of *Luciola mingrelica, Luciola cruciata* or *Luciola lateralis* luciferase; is different to the amino acid which appears in the corresponding wild type sequence and wherein the luciferase enzyme has increased thermostability as compared to an enzyme having the amino acid of the corresponding wild-type luciferase at this position.

**9.** A protein according to any one of the preceding claims wherein the amino acid residue corresponding to amino acid 354 of the *Photinus pyralis* luciferase (356 in *Luciola luciferase*) is mutated.

**10.** A protein according to claim 9 wherein the amino acid residue corresponding to residue 214 in *Photinus pyralis* luciferase or to residue 216 of *Luciola mingrelica*, *Luciola cruciata* or *Luciola lateralis* luciferase is mutated to a different hydrophobic amino acid consisting of the group isoleucine, leucine, valine, methionine, tryptophan, phenylalanine, proline, cysteine and alanine, in particular to isoleucine, leucine or valine.

**11.** A nucleic acid which encodes a luciferase according to any one of the preceding claims.

**12.** A nucleic acid according to claim 11 which comprises a synthetic gene.

**13.** A nucleic acid according to claim 11 wherein the codon usage has been optimised for a particular expression host and/or unique restriction sites have been introduced.

**14.** A nucleic acid according to claim 11 or claim 12 which comprises nucleotides 9-1661 of SEQ ID NO 1, or a sequence which has at least 90% similarity thereto.

**15.** A vector comprising a nucleic acid according to any one of claims 11 to 14.

**16.** A cell transformed with a vector according to claim 15.

**17.** A method of producing a protein according to any one of claims 1 to 10, which method comprises culture of a cell according to claim 16.

**18.** The use of a protein according to any one of claims 1 to 10 in a bioluminescent assay.

**19.** A kit comprising a protein according to any one of claims 1 to 10.

**20.** A kit according to claim 19 which further comprises luciferin.

**21.** An assay for determining the presence in a sample of coA, which assay comprises adding to a sample suspected of containing CoA, luciferase as claimed in any one of claims 1 to 10 above together with other reagents which are required to bring about a luciferase/luciferin reaction, measuring the wavelength of light emitted from the sample and relating this to the presence or absence of CoA.

**22.** An assay according to claim 21 for use in diagnosis of diabetes.

## Patentansprüche

**1.** Rekombinantes Protein mit Luciferase-Aktivität und einer Ähnlichkeit von mindestens 90 % mit einer Wildtyp-Luciferase von *Photinus pyralis*, *Luciola mingrelica*, *Luciola cruciata* oder *Luciola, lateralis, Hotaria parvula, Pyrophorus plagiophthalamus, Lampyris noctiluca, Pyrocoelia miyako* oder *Phrixothrix,* wobei in der Sequenz des Enzyms der Aminosäurerest, der Rest 357 in der Luciferase von *Photinus pyralis* entspricht, so mutiert ist, dass das Luciferase-Enzym befähigt ist, Licht bei einer im Vergleich zur entsprechenden Wildtyp-Luciferase unterschiedlichen Wellenlänge zu emittieren, und/oder im Vergleich zur entsprechenden Wildtyp-Luciferase erhöhte Thermostabilität aufweist, und wobei die Ähnlichkeit unter Anwendung eines Alignment-Algorithmus, wie er von Lipman und Paerson definiert wurde (1985, Science 227: 1435-1441), mit den Parametern ktup = 1, Gap-Penalty = 4 und Gap-Länge der Gap-Penalty = 12 bestimmt ist.

**2.** Rekombinantes Protein nach Anspruch 1, bei dem die Sequenz der Wildtyp-Luciferase die Sequenz der Luciferase von *Photinus pyralis*, *Luciola mingrelica*, *Luciola cruciata* oder *Luciola lateralis* ist.

**3.** Rekombinantes Protein nach Anspruch 1, bei dem die Sequenz der Wildtyp-Luciferase die Sequenz der Luciferase von *Photinus pyralis*, *Luciola mingrelica*, *Luciola cruciata oder Luciola lateralis*, *Hotaria parvula*, *Pyrophorus plagiophthalamus*, *Lampyris noctiluca* oder *Pyrocoelia miyako* ist und der Aminosäurerest, der Rest 357 in der Luciferase von *Photinus pyralis* entspricht, anders mutiert ist als zu Asparaginsäure oder Glutaminsäure.

**4.** Rekombinantes Protein nach einem der vorhergehenden Ansprüche, bei dem der Rest 357 in der Luciferase von *Photinus pyralis* entsprechende Aminosäurerest anders mutiert ist als zu Asparaginsäure, Glutaminsäure oder Valin.

**5.** Rekombinantes Protein nach Anspruch 1, bei dem die Sequenz von Wildtyp-Luciferase die Sequenz der Luciferase von *Phrixothrix* ist und der Rest 357 in der Luciferase von *Photinus pyralis* entsprechende Aminosäurerest anders mutiert ist als zu Leucin.

**6.** Rekombinantes Protein nach einem der vorhergehenden Ansprüche, bei dem der Rest 357 in der Luciferase von *Photinus pyralis* entsprechende Aminosäurerest zu Tyrosin, Phenylalanin, Tryptophan, Asparaginen, Glutamin, Serin oder Threonin mutiert ist.

**7.** Rekombinantes Protein nach Anspruch 6, bei dem der Rest 357 in der Luciferase von *Photinus pyralis* entsprechende Aminosäurerest zu Tyrosin mutiert ist.

**8.** Protein nach einem der vorhergehenden Ansprüche, das im Vergleich zu Wildtyp-Luciferase mindestens eine der folgenden Mutationen aufweist:

(a) Der Aminosäure 354 der Luciferase von *Photinus pyralis* oder Aminosäure 356 in der Luciferase von *Luciola* entsprechende Aminosäurerest ist mutiert;
(b) der Position 215 in der Luciferase von *Photinus pyralis* oder Position 217 in der Luciferase von *Luciola* entsprechende Aminosäurerest ist eine unterschiedliche hydrophobe Aminosäure,
oder
(c) der Rest 214 in der Luciferase von *Photinus pyralis* oder Rest 216 in der Luciferase von *Luciola mingrelica*, *Luciola cruciata* oder *Luciola lateralis* entsprechende Aminosäurerest,
(d) der Rest 232 in der Luciferase von *Photinus pyralis* oder Rest 234 in der Luciferase von *Luciola mingrelica*, *Luciola cruciata* oder *Luciola lateralis* entsrechende Aminosäurerest,
(e) der Rest 295 in der Luciferase von *Photinus pyralis* oder Rest 297 in der Luciferase von *Luciola mingrelica*, *Luciola cruciata* oder Luciola lateralis entsprechende Aminosäurerest,
(f) der Aminosäure 14 der Luciferase von *Photinus pyralis* oder Rest 16 der Luciferase von *Luciola mingrelica* oder Rest 17 der Luciferase von *Luciola cruciata* oder *Luciola lateralis* entsprechende Aminosäurerest,
(g) der Aminosäure 35 der Luciferase von *Photinus pyralis* oder Rest 37 der Luciferase von *Luciola mingrelica* oder Rest 38 der Luciferase von *Luciola cruciata* oder *Luciola lateralis* entsprechende Aminosäurerest,
(h) der dem Aminosäurerest 105 der Luciferase von *Photinus pyralis* oder Rest 106 der Luciferase von *Luciola mingrelica*, Rest 107 der Luciferase von *Luciola cruciata* oder von *Luciola lateralis* entsprechende Aminosäurerest,
(i) der dem Aminosäurerest 234 der Luciferase von *Photinus pyralis* oder Rest 236 der Luciferase von *Luciola mingrelica*, *Luciola cruciata* oder *Luciola lateralis* entsprechende Aminosäurerest,
(j) der dem Aminosäurerest 420 der Luciferase von *Photinus pyralis* oder Rest 422 der Luciferase von *Luciola mingrelica*, *Luciola cruciata* oder *Luciola lateralis* entsprechende Aminosäurerest,
(k) der dem Aminosäurerest 310 der Luciferase von *Photinus pyralis* oder Rest 312 der Luciferase von *Luciola mingrelica*, *Luciola cruciata* oder *Luciola lateralis* entsprechende Aminosäurerest

ist von der Aminosäure verschieden, die in der entsprechenden Wildtyp-Sequenz vorliegt,
und wobei das Luciferase-Enzym im Vergleich zu einem Enzym mit der Aminosäure der entsprechenden Wildtyp-Luciferase an dieser Position erhöhte Thermostabilität besitzt.

**9.** Protein nach einem der vorhergehenden Ansprüche, bei dem der Aminosäure 354 der Luciferase von *Photinus pyralis* (356 in der Luciferase von *Luciola*) entsprechende Aminosäurerest mutiert ist.

**10.** Protein nach Anspruch 9, bei dem der Rest 214 in der Luciferase von *Photinus pyralis* oder Rest 216 der Luciferase von *Luciola mingrelica*, *Luciola cruciata* oder *Luciola lateralis* entsprechende Aminosäurerest zu einer unterschiedlichen hydrophoben Aminosäure mutiert ist, die der Gruppe Isoleucin, Leucin, Valin, Methionin, Tryptophan, Phenylalanin, Prolin, Cystein und Alanin angehört, insbesondere zu Isoleucin, Leucin oder Valin.

**11.** Nucleinsäure, die eine Luciferase nach einem der vorhergehenden Ansprüche codiert.

**12.** Nucleinsäure nach Anspruch 11, die ein synthetisches Gen enthält.

**13.** Nucleinsäure nach Anspruch 11, bei der die Codon-Nutzung für einen speziellen Expressionswirt optimiert wurde und/oder nur einmal vorkommende Restriktionsstellen eingeführt wurden.

**14.** Nucleinsäure nach Anspruch 11 oder 12, welche die Nucleotide 9-1661 des Sequenzprotokolls SEQ ID NO 1 oder eine Sequenz enthält, die eine Ähnlichkeit von mindestens 90 % damit aufweist.

**15.** Vektor, der eine Nucleinsäure nach einem der Ansprüche 11 bis 14 enthält.

**16.** Zelle, die mit einem Vektor nach Anspruch 15 transformiert ist.

**17.** Verfahren zur Herstellung eines Proteins nach einem der Ansprüche 1 bis 10, das die Kultivierung einer Zelle nach Anspruch 16 umfasst.

**18.** Verwendung eines Proteins nach einem der Ansprüche 1 bis 10 in einem Biolumineszenz-Assay.

**19.** Kit, der ein Protein nach einem der Ansprüche 1 bis 10 enthält.

**20.** Kit nach Anspruch 19, der ferner Luciferin enthält.

**21.** Assay zur Bestimmung des Vorliegens von CoA in einer Probe, wobei das Assay umfasst:

Zugabe von Luciferase nach einem der obigen Ansprüche 1 bis 10 zusammen mit anderen Reagentien, die zur Durchführung einer Luciferase-/Luciferin-Reaktion erforderlich sind, zu einer Probe, von der zu vermuten ist, dass sie CoA enthält,
Messung der Wellenlänge des von der Probe emittierten Lichts und
Korrelieren der Messung mit dem Vorliegen oder Nichtvorliegen von CoA.

**22.** Assay nach Anspruch 21 zur Anwendung bei der Diagnose von Diabetes.

## Revendications

**1.** Protéine recombinée ayant une activité de luciférase et au moins 90% de similarité avec une luciférase de type sauvage de *Photinus pyralis, Luciola mingrelica*, *Luciola cruciata* ou *Luciola lateralis, Hotaria parvula*, *Pyrophorus plagiophthalamus, Lampyris noctiluca*, *Pyrocoelia miyako,* ou *Phrixothrix,* où dans la séquence de l'enzyme, le résidu acide aminé correspondant au résidu 357 dans la luciférase de *Photinus pyralis* est muté par comparaison à la luciférase de type sauvage correspondante, de sorte que l'enzyme luciférase est capable d'émettre de la lumière à une autre longueur d'onde par comparaison avec la luciférase de type sauvage correspondante et/ou a une thermostabilité augmentée par comparaison avec la luciférase de type sauvage correspondante, et où la similarité est déterminée à l'aide d'un algorithme d'alignement tel que défini par Lipman et Pearson (1985, Science 227 : 1435-1441) avec les paramètres ktup = 1, pénalité trou = 4 et longueur de pénalité trou = 12.

**2.** Protéine recombinée selon la revendication 1, dans laquelle la séquence de la luciférase de type sauvage est celle de la luciférase de *Photinus pyralis, Luciola mingrelica, Luciola cruciata* ou *Luciola lateralis.*

**3.** Protéine recombinée selon la revendication 1, dans laquelle ladite séquence de la luciférase de type sauvage est celle de la luciférase de *Photinus pyralis, Luciola mingrelica, Luciola cruciata* ou *Luciola lateralis*, *Hotaria parvula, Pyrophorus plagiophthalamus, Lampyris noctiluca* ou *Pyrocoelia miyako,* et le résidu acide aminé correspondant au résidu 357 dans la luciférase de *Photinus pyralis,* est muté en un autre acide aminé que l'acide aspartique ou l'acide glutamique.

**4.** Protéine recombinée selon l'une quelconque des revendications précédentes, dans laquelle le résidu acide aminé correspondant au résidu 357 dans la luciférase de *Photinus pyralis*, est muté en un autre acide aminé que l'acide aspartique, l'acide glutamique ou la valine.

**5.** Protéine recombinée selon la revendication 1, dans laquelle ladite séquence de la luciférase de type sauvage est celle de la luciférase de *Phrixothrix,* et le résidu acide aminé correspondant au résidu 357 dans la luciférase de *Photinus pyralis,* est muté en un autre acide aminé que la leucine.

**6.** Protéine recombinée selon l'une quelconque des revendications précédentes, dans laquelle le résidu acide aminé correspondant au résidu 357 dans la luciférase de *Photinus pyralis,* est muté en tyrosine, phénylalanine, tryptophane, asparagine, glutamine, sérine ou thréonine.

**7.** Protéine recombinée selon la revendication 6, dans laquelle le résidu acide aminé correspondant au résidu 357 dans la luciférase de *Photinus pyralis,* est muté en tyrosine.

**8.** Protéine selon l'une quelconque des revendications précédentes, qui a au moins une des mutations suivantes, par comparaison à la luciférase de type sauvage :

(a) le résidu acide aminé correspondant au résidu 354 dans la luciférase de *Photinus pyralis* à l'acide aminé 356 dans la luciférase de *Luciola* est muté ;
(b) le résidu acide aminé correspondant à la position 215 dans la luciférase de *Photinus pyralis* ou à la position 217 dans la luciférase de *Luciola* est un autre acide aminé hydrophobe ;
(c) le résidu acide aminé correspondant au résidu 214 dans la luciférase de *Photinus pyralis* ou au résidu 216 dans la luciférase de *Luciola mingrelica*, *Luciola cruciata* ou *Luciala lateralis* ;
(d) le résidu acide aminé correspondant au résidu 232 dans la luciférase de *Photinus pyralis* ou au résidu 234 dans la luciférase de *Luciola mingrelica, Luciola cruciata* ou *Luciala lateralis ;*
(e) le résidu acide aminé correspondant au résidu 295 dans la luciférase de *Photinus pyralis* ou au résidu 297 dans la luciférase de *Luciola mingrelica*, *Luciola cruciata* ou *Luciala lateralis* ;
(f) le résidu acide aminé correspondant au résidu 14 dans la luciférase de *Photinus pyralis* ou au résidu 16 dans la luciférase de *Luciola mingrelica*, ou 17 dans *Luciola cruciata* ou *Luciala lateralis* ;
(g) le résidu acide aminé correspondant au résidu 35 dans la luciférase de *Photinus pyralis* ou au résidu 37 dans la luciférase de *Luciola mingrelica*, ou au résidu 38 dans *Luciola cruciata* ou *Luciala lateralis* ;
(h) le résidu acide aminé correspondant au résidu acide aminé 105 dans la luciférase de *Photinus pyralis* ou au résidu 106 dans la luciférase de *Luciola mingrelica,* ou 107 dans *Luciola cruciata* ou *Luciala lateralis ;*
(i) le résidu acide aminé correspondant au résidu acide aminé 234 dans la luciférase de *Photinus pyralis* ou au résidu 236 dans la luciférase de *Luciola mingrelica, Luciola cruciata* ou *Luciala lateralis ;*
(j) le résidu acide aminé correspondant au résidu acide aminé 420 dans la luciférase de *Photinus pyralis* ou au résidu 422 dans la luciférase de *Luciola mingrelica, Luciola cruciata* ou *Luciala lateralis ;*
(k) le résidu acide aminé correspondant au résidu acide aminé 310 dans la luciférase de *Photinus pyralis* ou au résidu 312 dans la luciférase de *Luciola mingrelica, Luciola cruciata* ou *Luciala lateralis ;*

est différent de l'acide aminé qui est présent dans la séquence de type sauvage correspondante et où l'enzyme luciférase a une thermostabilité augmentée par comparaison à une enzyme ayant l'acide aminé de la luciférase de type sauvage correspondante en cette position.

**9.** Protéine selon l'une quelconque des revendications précédentes, dans laquelle le résidu acide aminé correspondant à l'acide aminé 354 dans la luciférase de *Photinus pyralis* (356 dans la luciférase de *Luciola)* est muté.

**10.** Protéine selon la revendication 9, dans laquelle le résidu acide aminé correspondant au résidu 214 dans la luciférase de *Photinus pyralis* ou au résidu 216dans la luciférase de *Luciola mingrelica*, *Luciola cruciata ou Luciola lateralis* est muté en un autre acide aminé hydrophobe, parmi le groupe consistant en isoleucine, leucine, valine, méthionine, tryptophane, phénylalanine, proline, cystéine et alanine, en particulier isoleucine, leucine ou valine.

**11.** Acide nucléique qui code une luciférase selon l'une quelconque des revendications précédentes.

**12.** Acide nucléique selon la revendication 11, qui comprend un gène synthétique.

**13.** Acide nucléique selon la revendication 11, dans lequel l'usage des codons a été optimalisé pour un hôte d'expression particulier et/ou des sites de restriction uniques ont été introduits.

**14.** Acide nucléique selon la revendication 11 ou 12, qui comprend les nucléotides 9-1661 de SEQ ID N°1, ou une séquence qui a au moins 90% de similarité avec celle-ci.

**15.** vecteur comprenant un acide nucléique selon l'une quelconque des revendications 11 à 14.

**16.** Cellule transformée avec un vecteur selon la revendication 15.

**17.** Procédé de production d'une protéine selon l'une quelconque des revendications 1 à 10, lequel procédé comprend la culture d'une cellule selon la revendication 16.

**18.** Utilisation d'une protéine selon l'une quelconque des revendications 1 à 10, dans un dosage bioluminescent.

**19.** Kit comprenant une protéine selon l'une quelconque des revendications 1 à 10.

**20.** Kit selon la revendication 19, qui comprend en outre, la luciférine.

**21.** Dosage pour déterminer la présence de CoA dans un échantillon, lequel dosage comprend l'addition à un échantillon suspecté de contenir du CoA, de la luciférase selon l'une quelconque des revendications 1 à 10, avec d'autres réactifs qui sont nécessaires pour entraîner la réaction luciférase/luciférine, la mesure de la longueur d'onde de la lumière émise par l'échantillon et la relation de ceci avec la présence ou l'absence de CoA.

**22.** Dosage selon la revendication 21, à utiliser pour le diagnostic du diabète.

Fig.1.

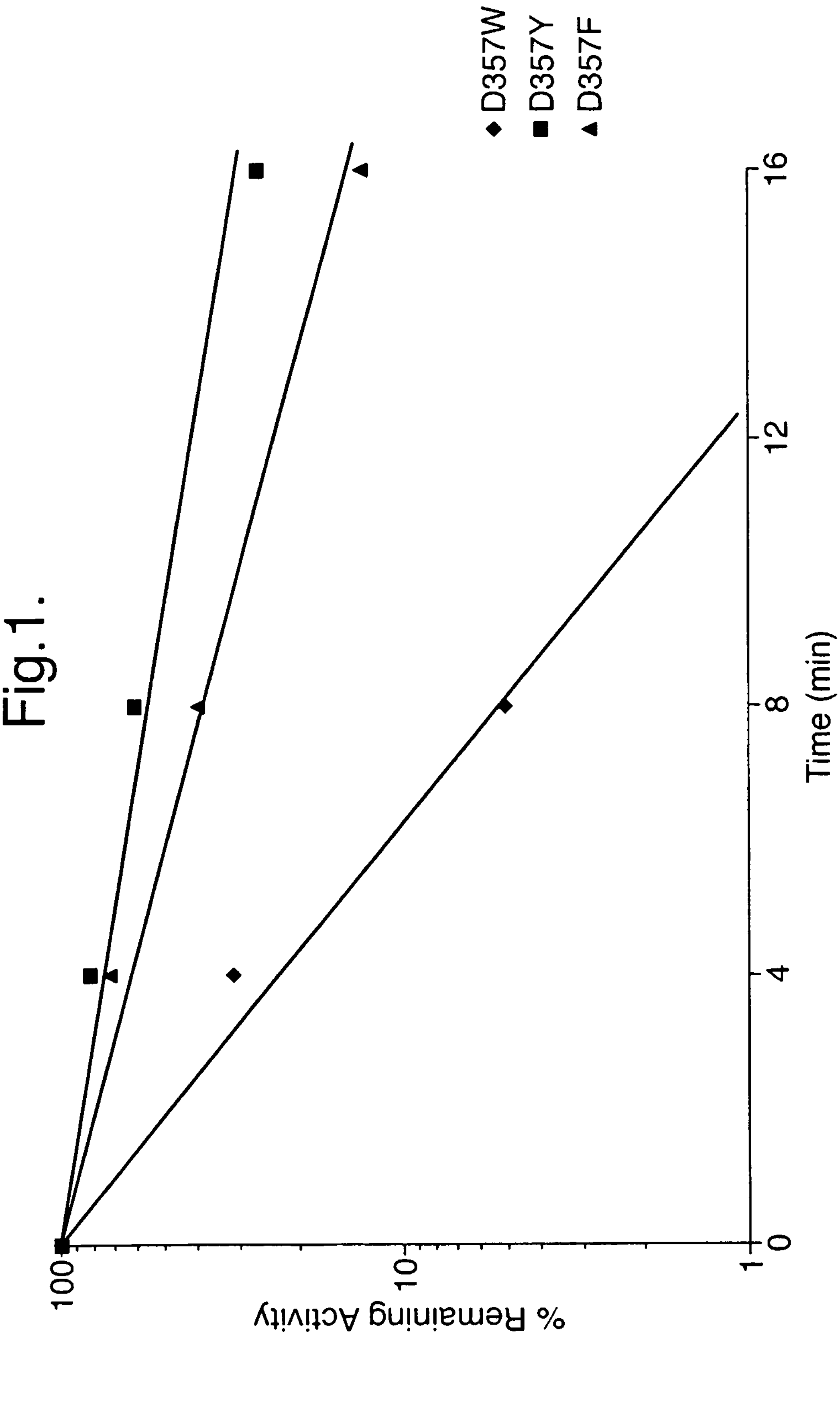
% Remaining Activity
100
10
1
Time (min)
0
4
8
12
16
D357W
D357Y
D357F

Fig.2(a).

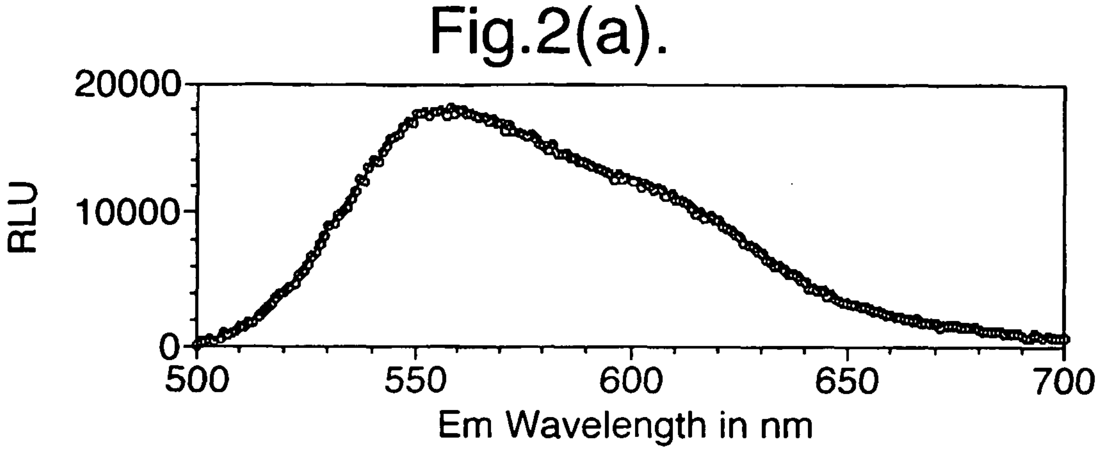


Fig.2(b).

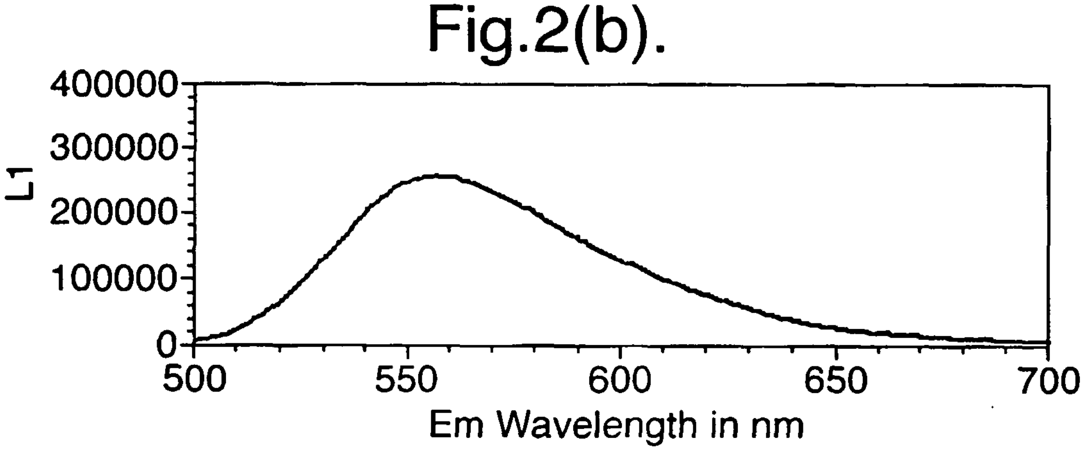


Fig.2(c).

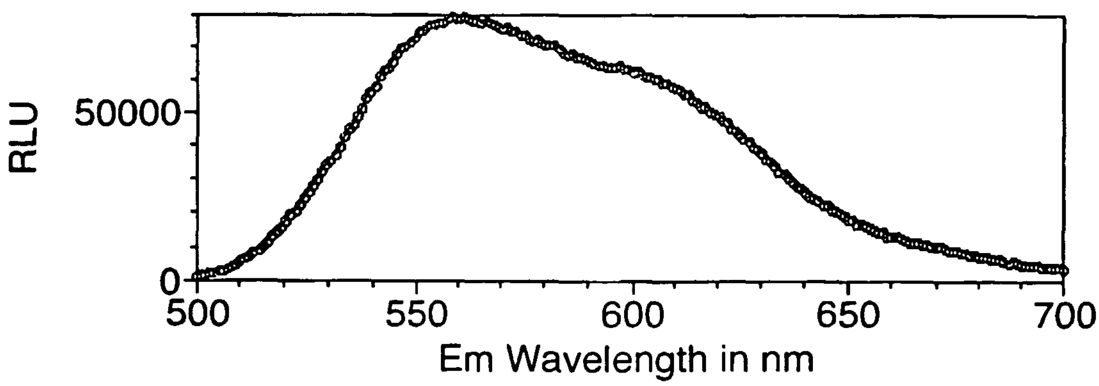

Fig.2(d).

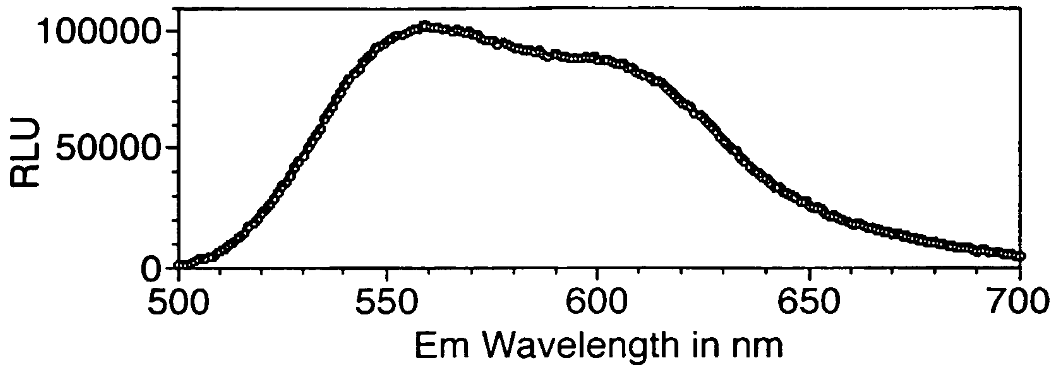


Fig.2(e).

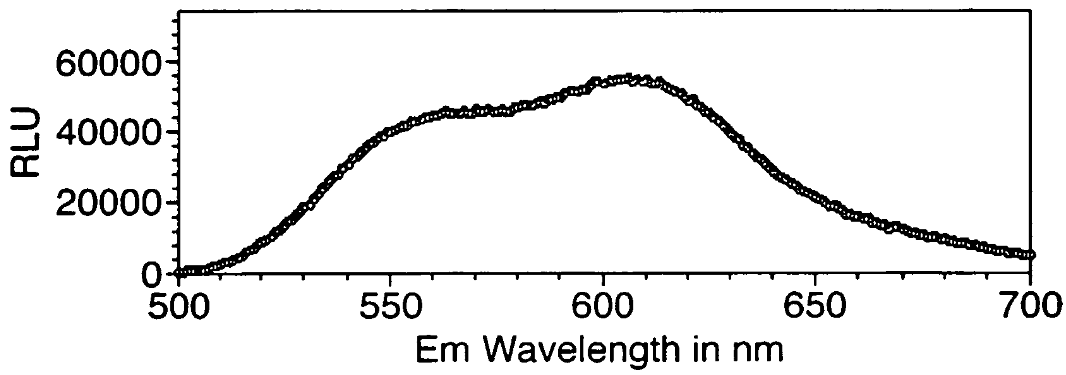


Fig.2(f).

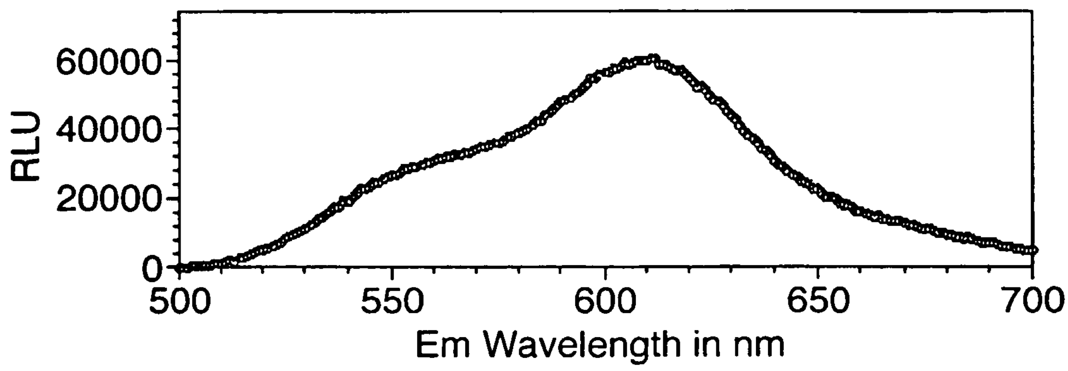

Fig.2(g).

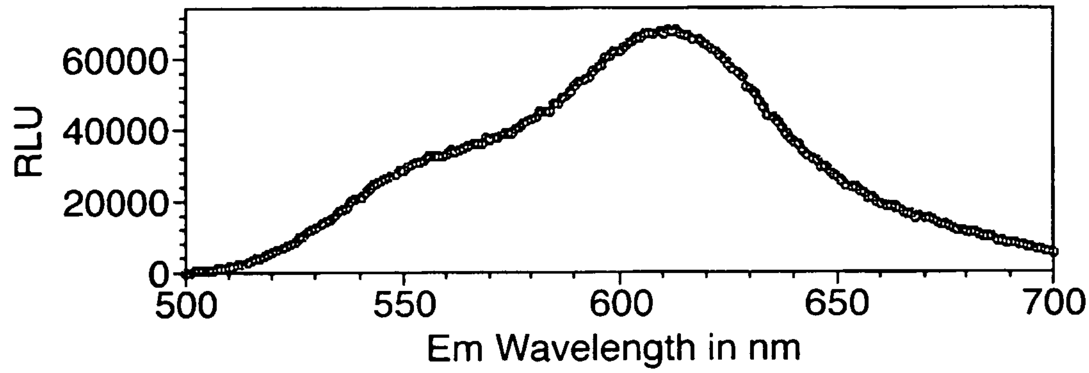
RLU
60000
40000
20000
0
500
550
600
650
700
Em Wavelength in nm

Fig.2(h).

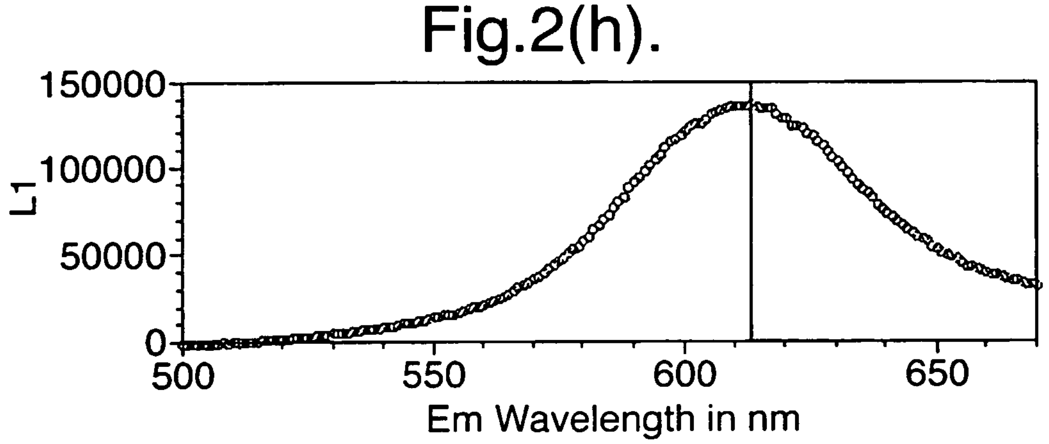
L1
150000
100000
50000
0
500
550
600
650
Em Wavelength in nm

Fig.3.

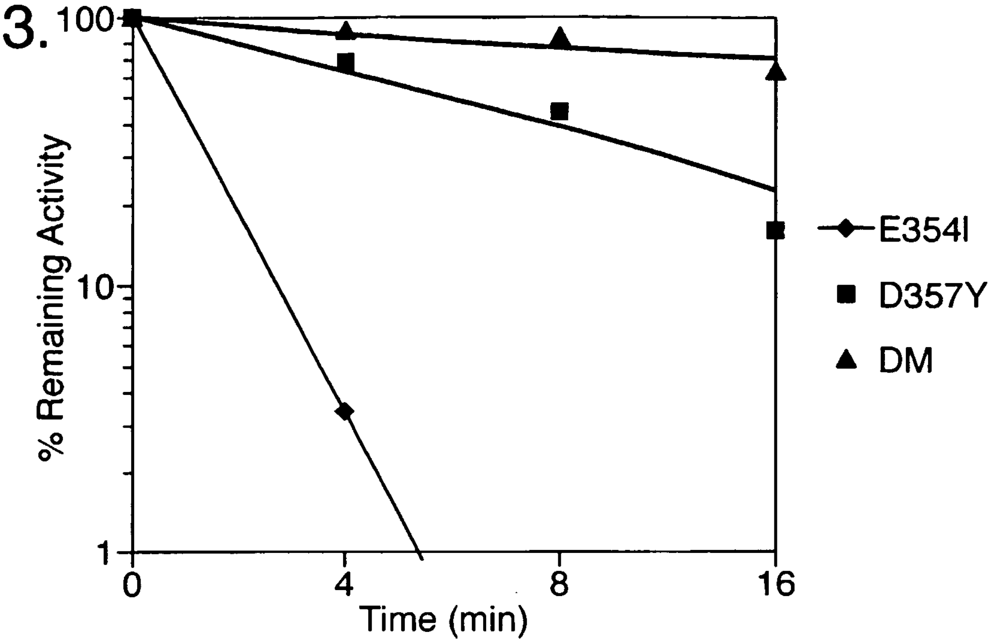


Fig.4(a).

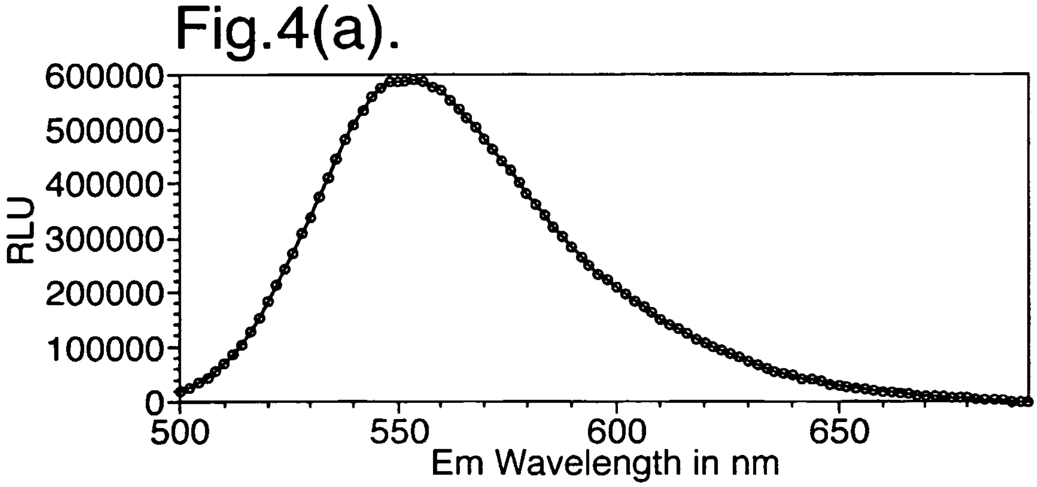


Fig.4(b).

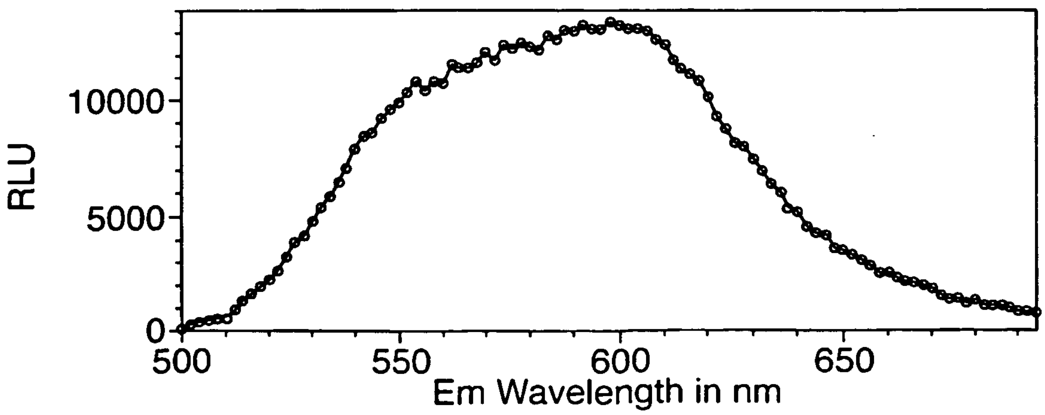

Fig.5.

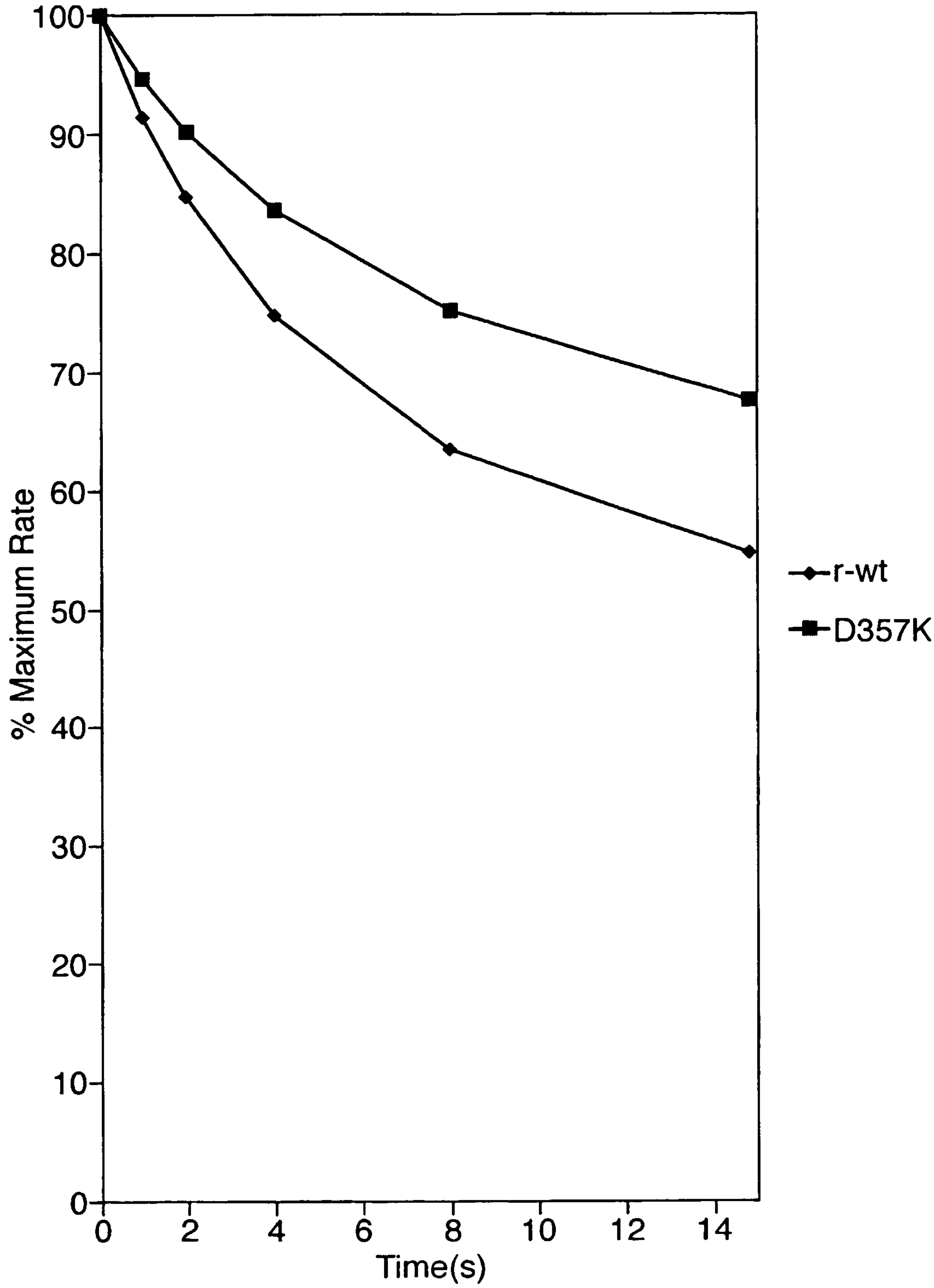
100
90
80
70
60
50
40
30
20
10
0
% Maximum Rate
0
2
4
6
8
10
12
14
Time(s)
r-wt
D357K

Fig.6.

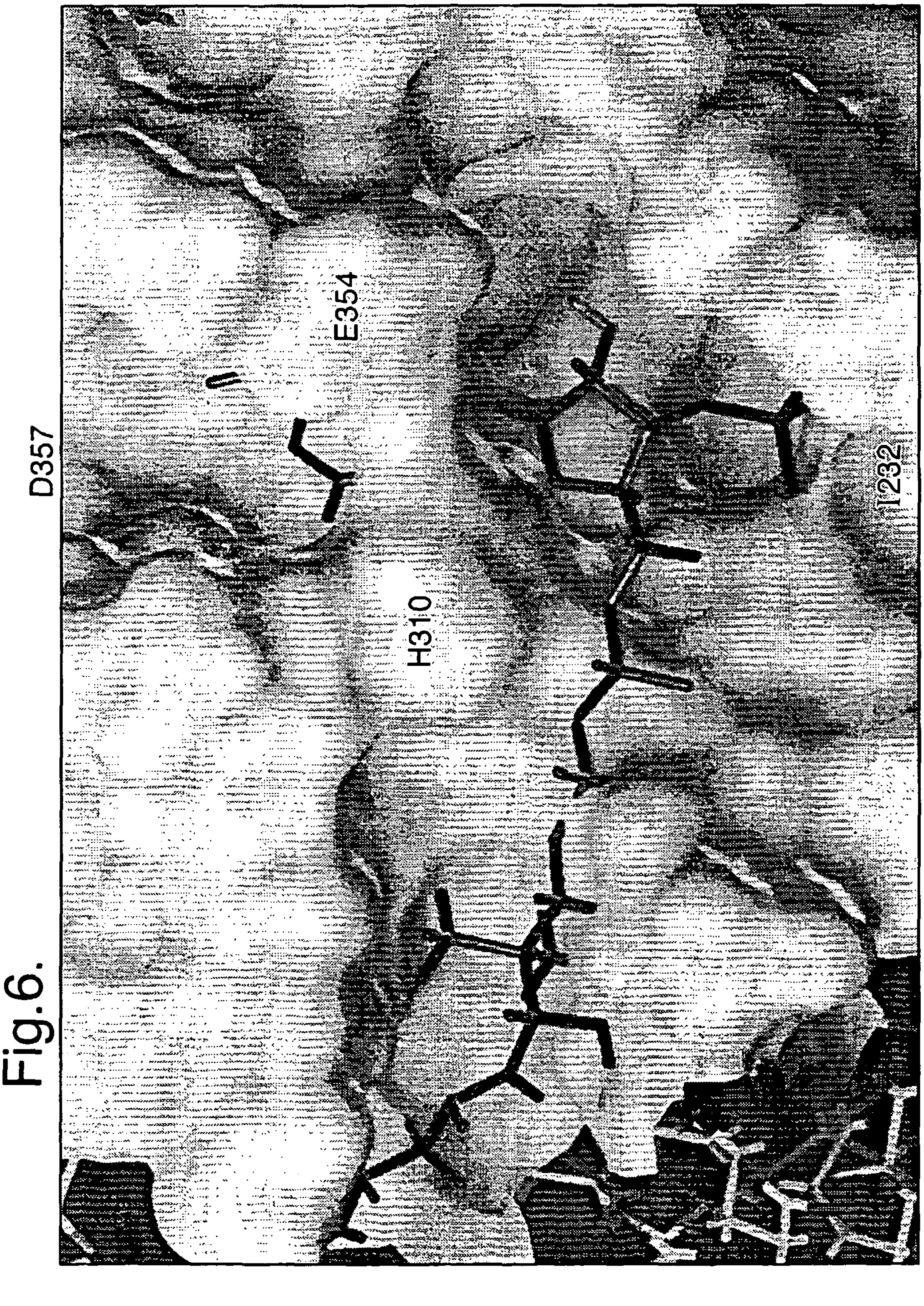

Fig.7a.

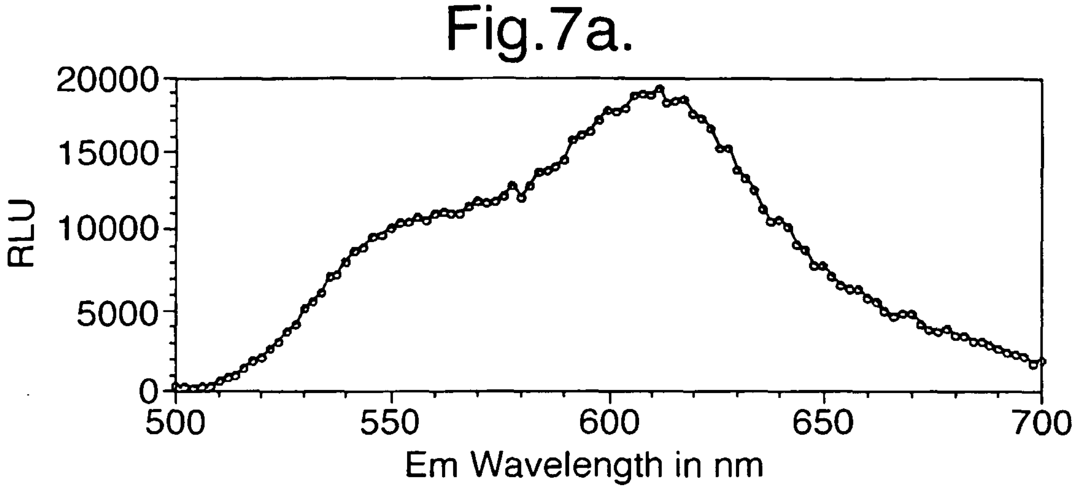


Fig.7b.

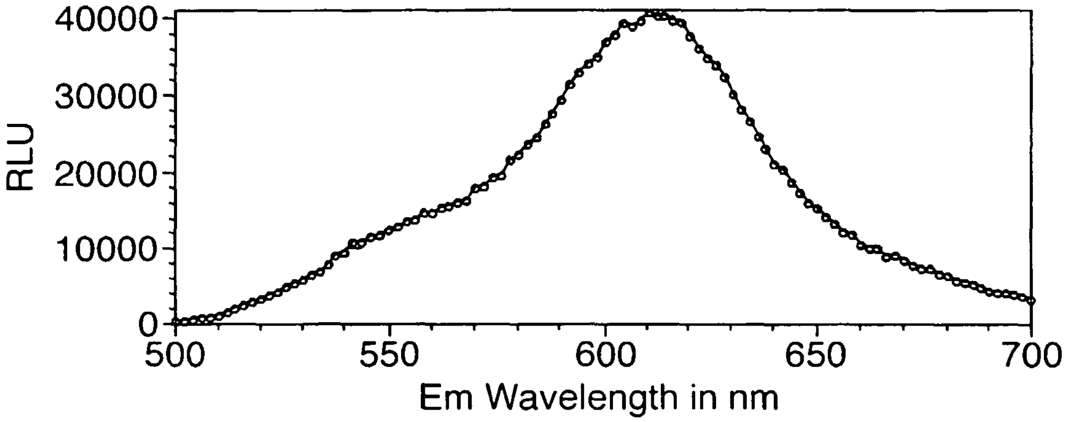


Fig.7c.

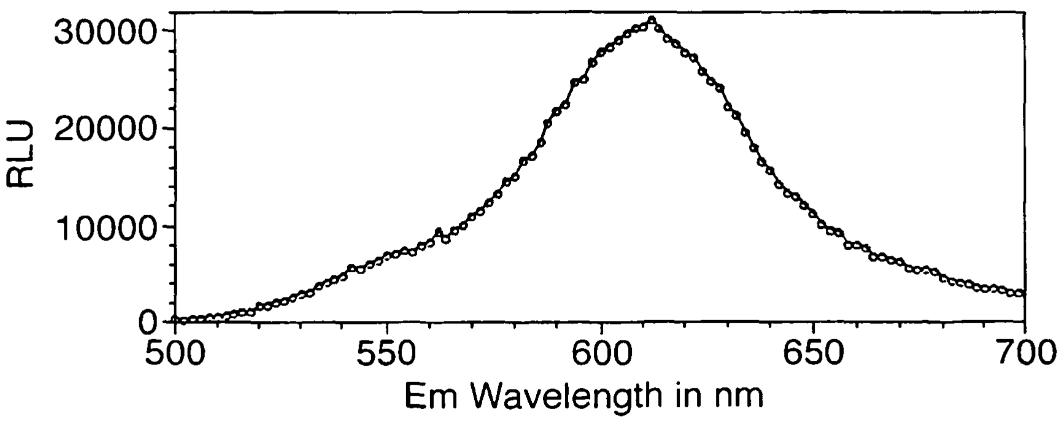

Fig.8(a).

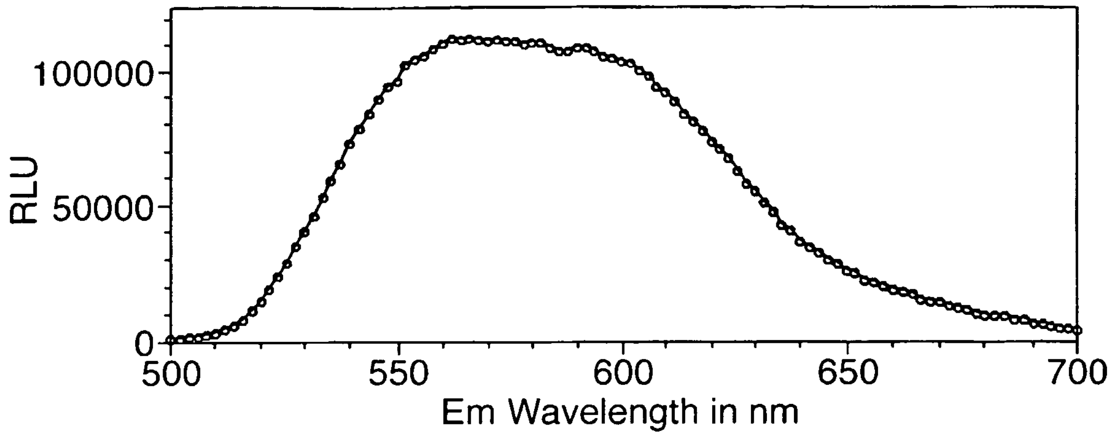


Fig.8(b).

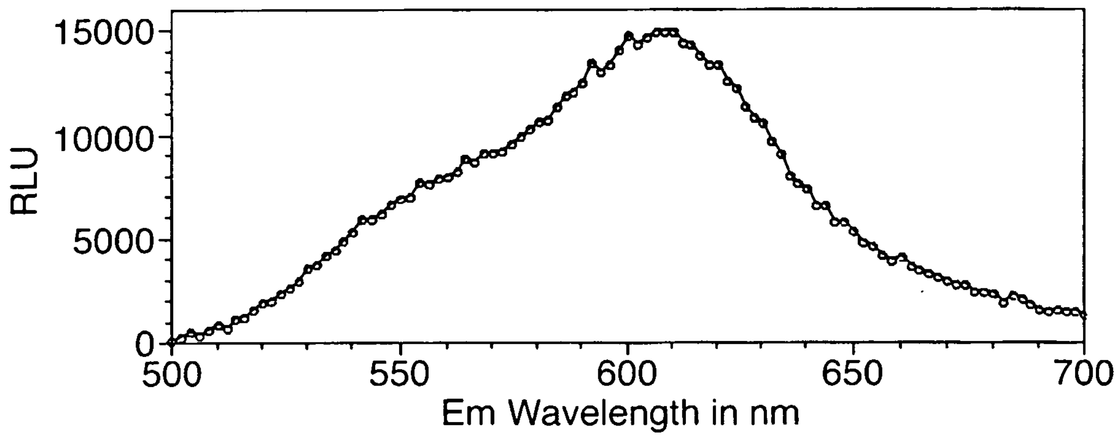


Fig.8(c).

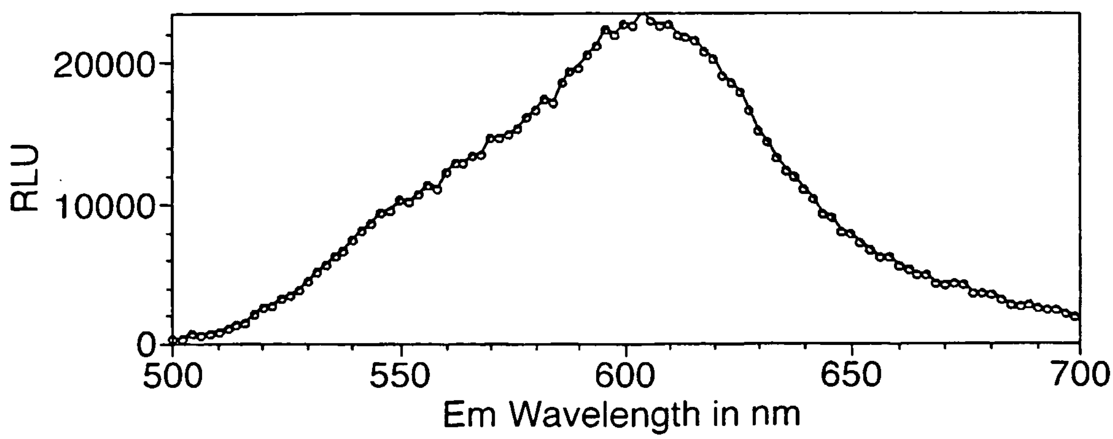

Fig.9.

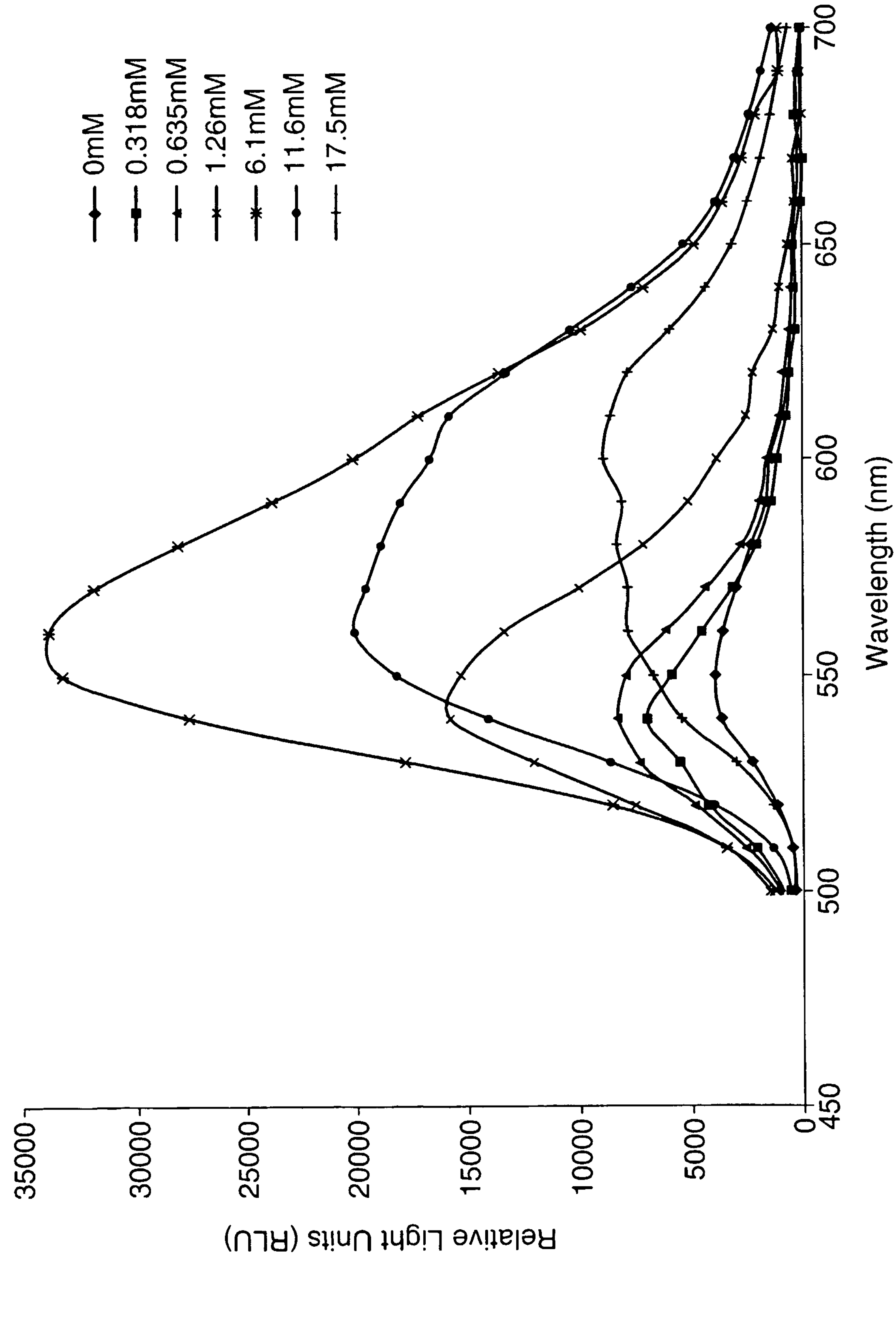
0mM
0.318mM
0.635mM
1.26mM
6.1mM
11.6mM
17.5mM
Relative Light Units (RLU)
35000
30000
25000
20000
15000
10000
5000
0
450
500
550
600
650
700
Wavelength (nm)

Fig.10.

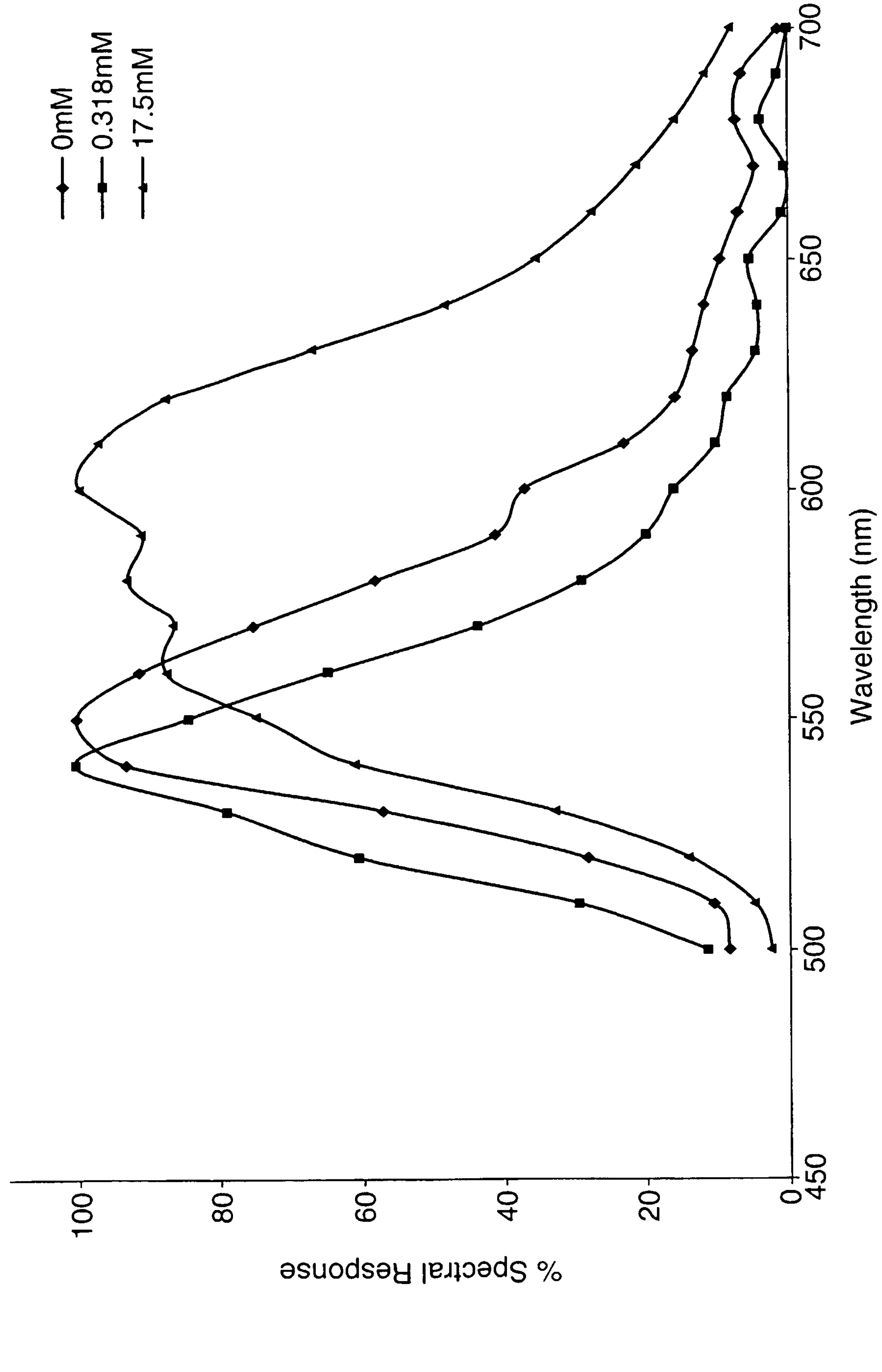
0mM
0.318mM
17.5mM
% Spectral Response
0
20
40
60
80
100
450
500
550
600
650
700
Wavelength (nm)

Fig.11(a).

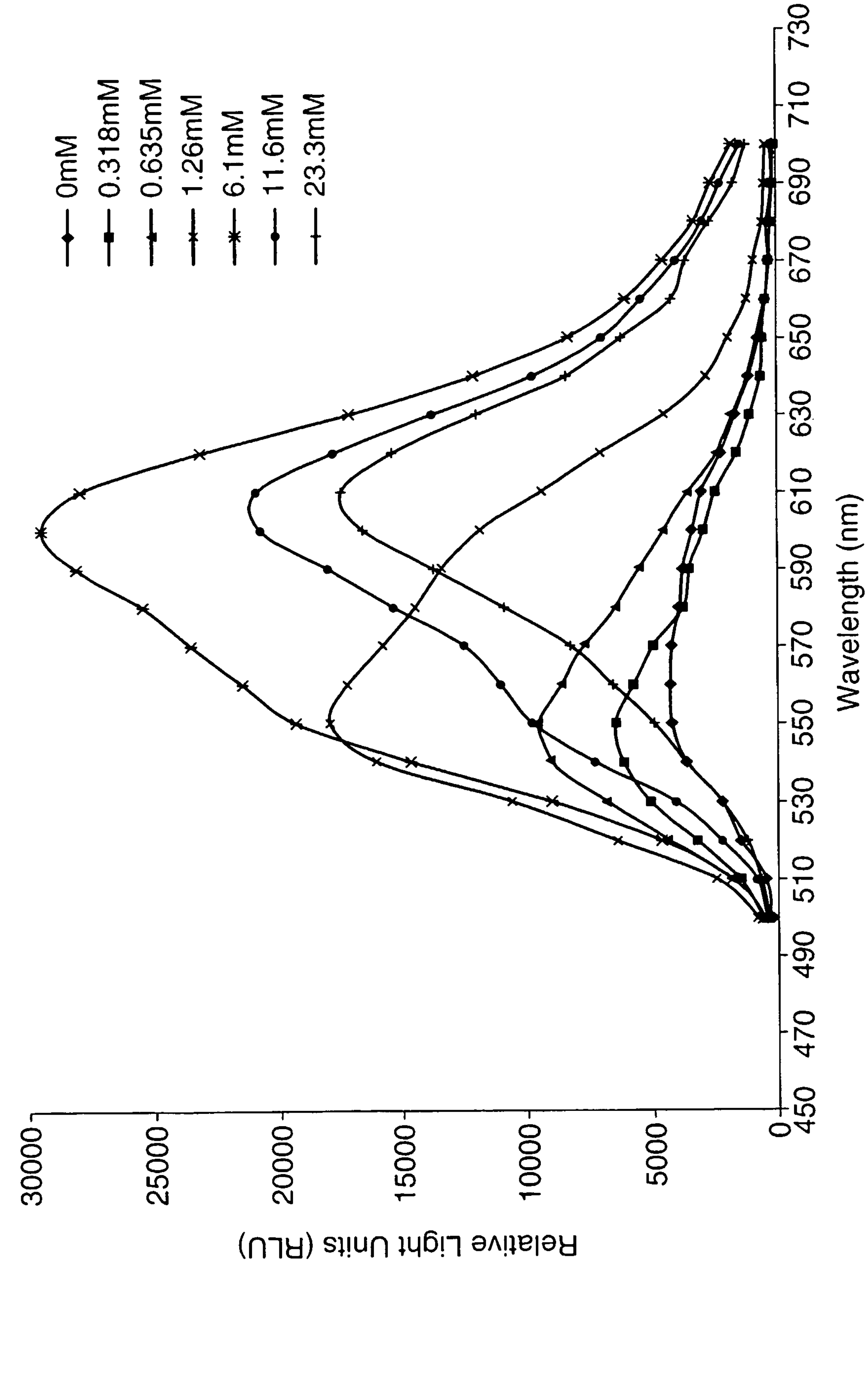
0mM
0.318mM
0.635mM
1.26mM
6.1mM
11.6mM
23.3mM
Relative Light Units (RLU)
30000
25000
20000
15000
10000
5000
0
450
470
490
510
530
550
570
590
610
630
650
670
690
710
730
Wavelength (nm)

Fig.11(b).

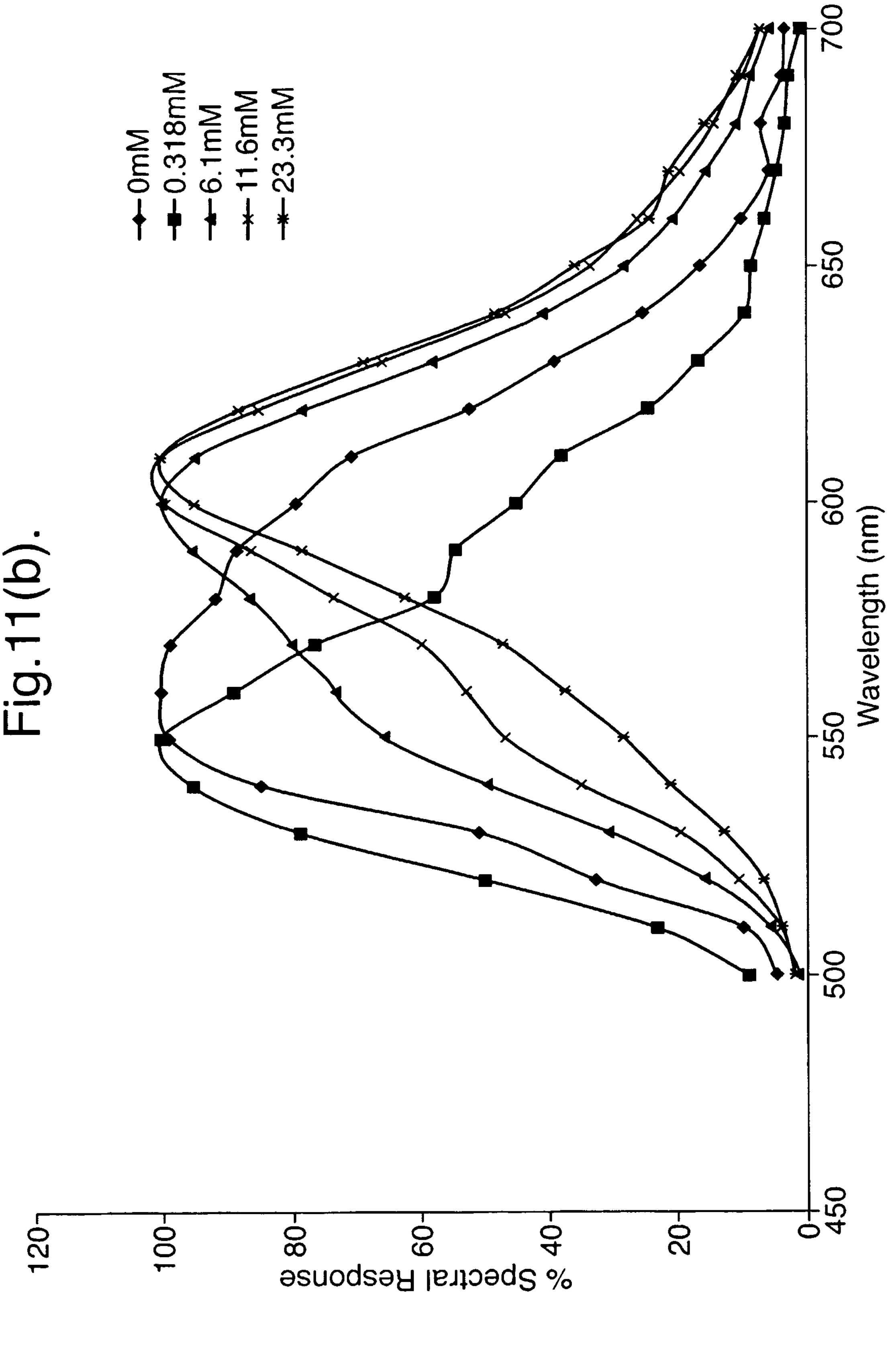
0mM
0.318mM
6.1mM
11.6mM
23.3mM
% Spectral Response
120
100
80
60
40
20
0
450
500
550
600
650
700
Wavelength (nm)

Fig.12.

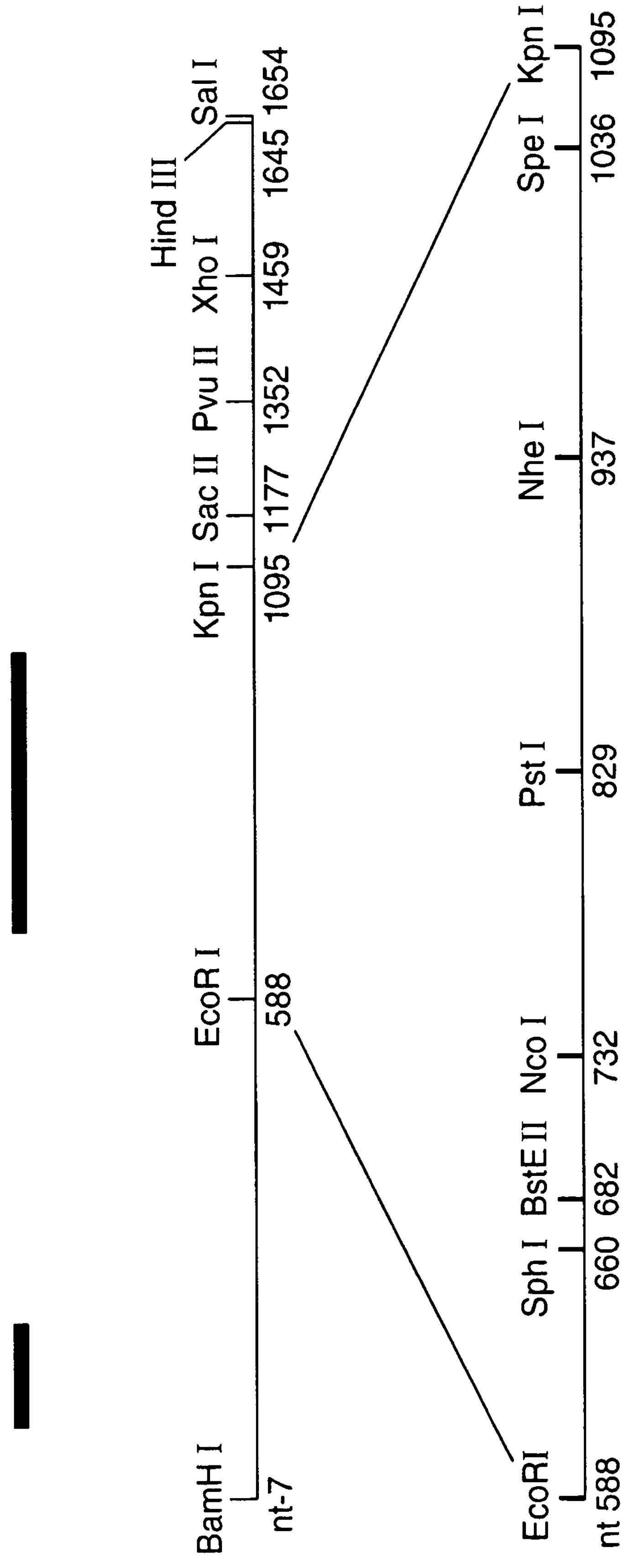
BamH I
nt-7
EcoR I
588
Kpn I
1095
Sac II
1177
Pvu II
1352
Xho I
1459
Hind III
1645
Sal I
1654
EcoRI
nt 588
Sph I
660
BstE II
682
Nco I
732
Pst I
829
Nhe I
937
Spe I
1036
Kpn I
1095

# Fig.13.

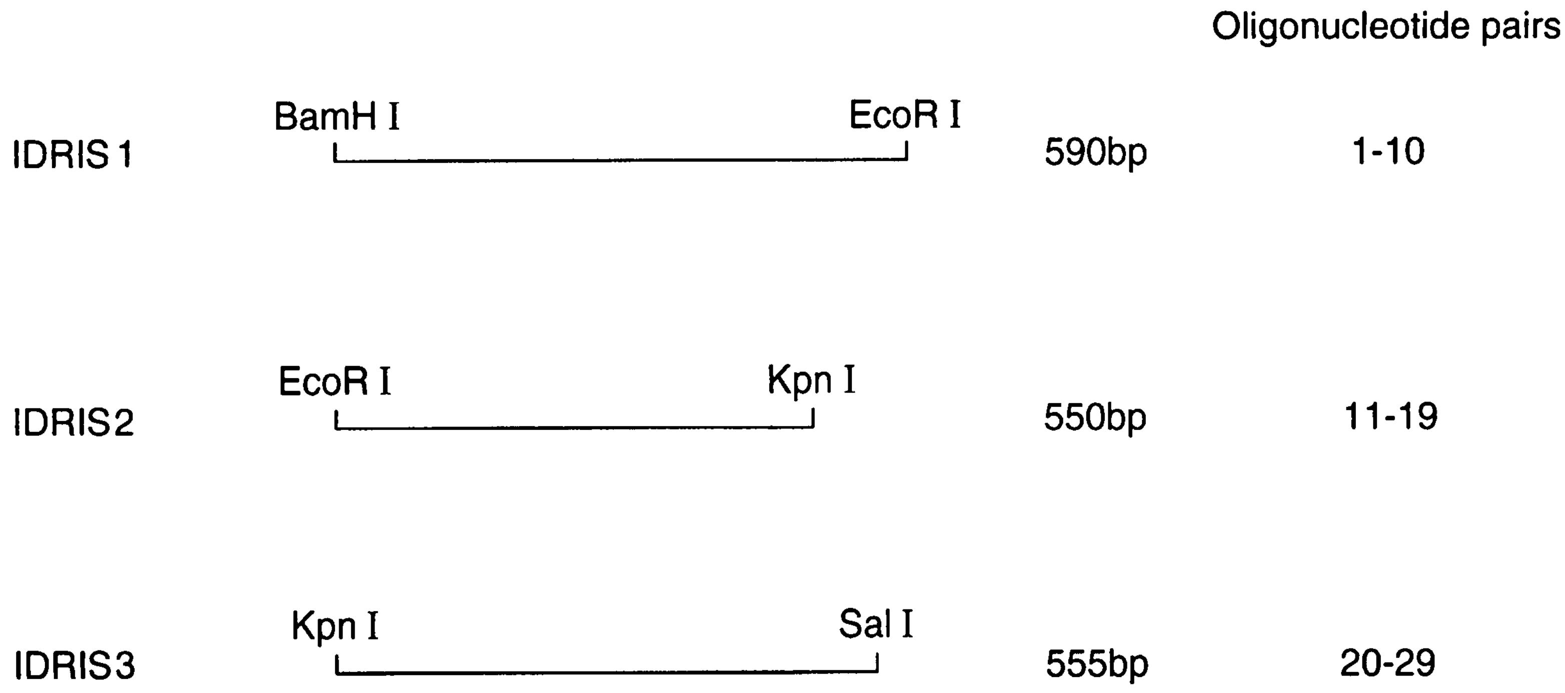
Oligonucleotide pairs
IDRIS1
BamH I
EcoR I
590bp
1-10
IDRIS2
EcoR I
Kpn I
550bp
11-19
IDRIS3
Kpn I
Sal I
555bp
20-29

Fig.14.(1/8)

```
                                                      BbeI
                                                     HaeII
                                                     HhaI|
                                                   NlaIV||
                                                    SfoI||
                                                  BsaHI|||
                                                 HinP1I|||
                                                   NarI|||
                                                  BanI||||
                                                  KasI||||
                                               HpaII |||||
                                                NciI |||||
       DpnI                                    ScrFI |||||
      NlaIV                                  BssKI | |||||
     BamHI |                                 CviJI | |||||
     BstYI |               BbsI HgaI        HaeIII | |||||
      MboI |  AlwI   BsaHI   |MboII       Sau96I|  | |||||       MnlI
         | |     |       |  |    |             || | |||||          |
         ggatccaaatggaagacgccaaaaacatcaagaaaggcccggcgccattctatcctctgg
       1 ---------+---------+---------+---------+---------+---------+ 60
         cctaggtttaccttctgcggtttttgtagttctttccgggccgcggtaagataggagacc

c      (I)(Q) M  E  D  A  K  N  I  K  K  G  P  A  P  F  Y  P  L  E -
              ↑
                    BpmI
                   Cac8I                                       BstNI
                  FokI  |                                      ScrFI
                Cac8I|  |                                    BssKI |
                 MwoI||  |                                   PspGI |
              MspA1I|||  |                                  NlaIV| |
                AciI ||||  |                             BstNI   || |
            BstF5I | ||||  |                             ScrFI   || |
             NlaIV | ||||  |                            BsaJI |  || |
             BanI | | ||||  |                           BssKI |  || |
         MnlI | | | ||||  |  HpyCH4V  CviJI    MaeIII   PspGI |  || |
            | | | | ||||  |        |      |        |       | |  || |
         aggatggcaccgctggcgagcaactgcataaggctatgaagcgttacgccctggttcctg
      61 ---------+---------+---------+---------+---------+---------+ 120
         tcctaccgtggcgaccgctcgttgacgtattccgatacttcgcaatgcgggaccaaggac

c          D  G  T  A  G  E  Q  L  H  K  A  M  K  R  Y  A  L  V  P  G -

                                                         AciI
                                                        BstUI
                                                     RsaI |
                                                     TaiI |
```

# Fig.14 (Cont). (2/8)

```
          SfaNI                                          Csp6I|  |
       MfeI    |                                          HphI|  |
   Tsp509I     |                                        BsaAI||  |
      RsaI |   |              MnlI                      BsiWI||  |        BstBI
    Csp6I| |   |          HpyCH4V | TaqI               MaeII|||  |         TaqI
         || |   |                | |    |                  ||||  |            |
     gtacaattgcttttacagatgcacatatcgaggtgaacatcacgtacgcggaatacttcg
 121 ---------+---------+---------+---------+---------+---------+ 180
     catgttaacgaaaatgtctacgtgtatagctccacttgtagtgcatgcgccttatgaagc

c      T  I  A  F  T  D  A  H  I  E  V  N  I  T  Y  A  E  Y  F  E -

               BstNI
               ScrFI
             BssKI |     AluI               HphI
             PspGI |     CviJI          Hpy188I |     Bst4CI
                 | |         |                | |          |
     aaatgtccgttcgcctggcagaagctatgaaacgctatggtctgaatacaaatcaccgta
 181 ---------+---------+---------+---------+---------+---------+ 240
     tttacaggcaagcggaccgtcttcgatactttgcgataccagacttatgtttagtggcat

c      M  S  V  R  L  A  E  A  M  K  R  Y  G  L  N  T  N  H  R  I -

                                                          HhaI
                                                       BstUI |
                                                       Cac8I |
                                                        HhaI |
                                                     HinP1I |
                                                       MwoI| |
                    BtsI                             BssHII|| |
        HpyCH4V  TspRI         EarI       HpaII     HinP1I|| |
     Hpy99I   |MboII |      Tsp509I|     BsrFI|    BslI   ||| |
           |   |    | |            ||         ||      |    ||| |
     tcgtcgtatgcagtgaaaactctcttcaattctttatgccggtgctgggcgcgcttttta
 241 ---------+---------+---------+---------+---------+---------+ 300
     agcagcatacgtcacttttgagagaagttaagaaatacggccacgacccgcgcgaaaaat

c      V  V  C  S  E  N  S  L  Q  F  F  M  P  V  L  G  A  L  F  I -

                Cac8I
                 NaeI
               HpaII|
              BsrFI||
               HhaI||
             NgoMIV||
              SgrAI||
           HinP1I |||                        TaiI        Bst4CI
       HpyCH4V   |  |||           PsiI  MaeII  |     MseI    |
             |     |  |||              |      |  |        |    |
     tcggtgttgcagttgcgccggcgaacgacatttataatgaacgtgaactgcttaacagta
 301 ---------+---------+---------+---------+---------+---------+ 360
     agccacaacgtcaacgcggccgcttgctgtaaatattacttgcacttgacgaattgtcat

c      G  V  A  V  A  P  A  N  D  I  Y  N  E  R  E  L  L  N  S  M -

                                                         ApoI
                                                      Tsp509I
```

# Fig.14 (Cont).(3/8)

```
                                                       HpyCH4V      |
                                                        Cac8I |     |
                                                      CviJI | |     |
                CviJI                               HaeIII | |     |
            Fnu4HI |     BbvI                    Eco0109I | | |     |
              TseI| |Bst4CI |                       Sau96I | | |     |
                  || |     | |                            | | | |     |
      tgaacatttcgcagcctaccgtagtctttgtttccaaaaagggcctgcaaaaaattctca
 361  ---------+---------+---------+---------+---------+---------+ 420
      acttgtaaagcgtcggatggcatcagaaacaaaggtttttcccggacgttttttaagagt

c      N  I  S  Q  P  T  V  V  F  V  S  K  K  G  L  Q  K  I  L  N -

  HpyCH4V                                            HinfI
     TaiI|                                              TfiI
 MaeII  ||        Tsp509I     Tsp509I          NlaIII|
     |  ||              |           |               ||
      acgtgcaaaaaaaactgccaattatccagaaaattattatcatggattctaaaacggatt
 421  ---------+---------+---------+---------+---------+---------+ 480
      tgcacgttttttttgacggttaataggtcttttaataatagtacctaagattttgcctaa

c      V  Q  K  K  L  P  I  I  Q  K  I  I  I  M  D  S  K  T  D  Y -

                                     MaeIII
                                     Tsp45I
                                     TaiI |
                                 MaeII  | |
        CviJI                 AflIII |  | |
  BstNI     |                   RsaI| |  | |
  ScrFI     |                 Csp6I|| |  | |                NciI
 BsaJI|     |                BsrGI||| |  | |                ScrFI
 BssKI||    |                 TatI||| |  | |              HpaII|   MnlI
 PspGI||    |          TaqI      |||| |  | |             BssKI||   MseI
      |||   |             |      |||| |  | |                  |||     |
      accagggctttcagtcgatgtacacgttcgtcacatctcatctgcctccgggttttaatg
 481  ---------+---------+---------+---------+---------+---------+ 540
      tggtcccgaaagtcagctacatgtgcaagcagtgtagagtagacggaggcccaaaattac

c      Q  G  F  Q  S  M  Y  T  F  V  T  S  H  L  P  P  G  F  N  E -

                                                                ApoI
                                                               EcoRI
                                                              NlaIII
                                   MaeIII                    Tsp509I
                                   Tsp45I                  BsphI   |
                                   DpnI |                  TspRI   |
                                  MlyI| |        HpyCH4V   DpnI|   |
            RsaI                 MboI|| |      MfeI      |BclI ||   |
          Csp6I|HinfI            PleI|| |   Tsp509I      |MboI ||   |
               ||    |              ||| |          |      |    | ||   |
      aatacgattttgtaccagagtcctttgatcgtgacaaaacaattgcactgatcatgaatt
 541  ---------+---------+---------+---------+---------+---------+ 600
      ttatgctaaaacatggtctcaggaaactagcactgttttgttaacgtgactagtactta a

c      Y  D  F  V  P  E  S  F  D  R  D  K  T  I  A  L  I  M  N  S -

                                                          Cac8I
```

# Fig.14 (Cont). (4/8)

```
                                         CviJI              SfaNI   |
                MnlI       Bsu36I       HaeIII          MslI     |  |
         CviJI    |     BsrI DdeI     Sau96I|  AciI HgaI  |      |  |
             |    |        |    |          ||     |    |  |      |  |
       cctctggctctactggtctgcctaagggtgtggcccttccgcatcgttgtgcctgcgtcc
   601 ---------+---------+---------+---------+---------+---------+ 660
       ggagaccgagatgaccagacggattcccacaccgggaaggcgtagcaacacggacgcagg

c        S  G  S  T  G  L  P  K  G  V  A  L  P  H  R  C  A  C  V  R -

                      FauI
                    DpnI |
                  MboI | |
                BstUI| | |
               AciI || | |
              Cac8I || | |
             AlwI | || | |
           NlaIII | || | |                   HpaII
             NspI | || | |   BstEII        BsaWI|         DdeI
             SphI | || | |   MaeIII        BspEI|      HinfI |
          Cac8I | | || | |   BslI  |     BciVI ||BseMII TfiI |
              | | | || | |      |  |         | ||     |    | |
       gtttctcgcatgcccgcgatcctatttttggtaaccaaatcattccggatactgcgattc
   661 ---------+---------+---------+---------+---------+---------+ 720
       caaagagcgtacgggcgctaggataaaaaccattggtttagtaaggcctatgacgctaag

c        F  S  H  A  R  D  P  I  F  G  N  Q  I  I  P  D  T  A  I  L -

                          BstXI
                      NlaIII |
                        XcmI |
                     BsaJI | |
                      BtgI | |                        DpnI
                      NcoI | |  NlaIII         Hpy188I |
Hpy188I               StyI | |    NspI       CviJI  MboI |
      |                  | | |       |           |     | |
       tgagtgttgttccattccaccatggtttggcatgtttactacactcggctatctgatct
   721 ---------+---------+---------+---------+---------+---------+ 780
       actcacaacaaggtaaggtggtaccaaaccgtacaaatgatgtgagccgatagactaga

c        S  V  V  P  F  H  H  G  F  G  M  F  T  T  L  G  Y  L  I  C -

                                                       HhaI
                                                   HinP1I |
                                                  MboII | |
                                                  MwoI| | |
                              EarI           MboII || | |HpyCH4V
                          MnlI   |      AluI     | || | | SfcI |
     CviJI             NlaIII |SapI    CviJI     | || | |MwoI| |
         |                  | |  |         |     | || | |   || |
       gtggctttcgtgtcgtcctcatgtatcgctttgaagaagagctgtttctgcgctccctgc
   781 ---------+---------+---------+---------+---------+---------+ 840
       caccgaaagcacagcaggagtacatagcgaaacttcttctcgacaaagacgcgagggacg

c        G  F  R  V  V  L  M  Y  R  F  E  E  E  L  F  L  R  S  L  Q -

                             NlaIV
```

# Fig.14 (Cont).(5/8)

```
 PstI      ApoI         HhaI  BanI |
 SbfI  Tsp509I      HinP1I |  MwoI |      MboII
   |        |            | |     | |        |
   aggattacaaaattcaaagtgcgcttctggtgccaaccctgtttcattcttcgccaaaa
841 ---------+---------+---------+---------+---------+---------+ 900
   tcctaatgttttaagtttcacgcgaagaccacggttgggacaaaagtaagaagcggtttt

c   D  Y  K  I  Q  S  A  L  L  V  P  T  L  F  S  F  F  A  K  S -

                                                        BseSI
                                                      BsiHKAI
                                                     Bsp1286I
                                                   HpyCH4V |
                                                   ApaLI | |
                                                 AciI  | | |
                                             Fnu4HI |  | | |
                                              AciI| |  | | |
                                            Cac8I || |  | | |
                          MboII             BcgI| || |  | | |
                         DpnI |             BfaI| || |  | | |
 Hpy188I               MboI | |     Tsp509I NheI|| || |  | | |
     |                    | | |          |    ||| || |  | | |
   gcactctgattgacaaatacgatctgtctaatcttcacgaaattgctagcggcggtgcac
901 ---------+---------+---------+---------+---------+---------+ 960
   cgtgagactaactgtttatgctagacagattagaagtgctttaacgatcgccgccacgtg

c   T  L  I  D  K  Y  D  L  S  N  L  H  E  I  A  S  G  G  A  P -
                                                     '315
                                                    BstF5I
                                                  BstNI |
       MnlI                                       ScrFI |
  BstBI  |       BcgI  HpyCH4V      FokI     BssKI |  |
   TaqI  | Hpy188I |AciI    | MboII  |      PspGI |  |
      |  |       | |   |    |    |  |          | |  |
   ctctttcgaaagaagtcggagaagcggttgcaaaacgcttccatcttccaggcatccgtc
961 ---------+---------+---------+---------+---------+---------+ 1020
   gagaaagctttcttcagcctcttcgccaacgttttgcgaaggtagaaggtccgtaggcag

c   L  S  K  E  V  G  E  A  V  A  K  R  F  H  L  P  G [I  R] Q -

                      TspRI
                   DdeI  |
   BseMII         BsaI|  |
  CviJI |        BsmAI|  | BfaI
 SfaNI  |  |BsmAI   ||  |SpeI|      Hpy188I
   |  |  |    |    ||  |   ||          |
   aaggctatggtctcactgagactactagtgctattctgattacaccgaagggcgatttca
1021 ---------+---------+---------+---------+---------+---------+ 1080
   ttccgataccagagtgactctgatgatcacgataagactaatgtggcttcccgctaaagt

c   G  Y  G  L  T  E  T  T  S  A  I  L  I  T  P  K  G  D  F  K -
                          |
                         345
          BsiEI
       AciI  |
      BstUI  |
       HhaI  |            KpnI
    HinP1I | |           NlaIV |
```

# Fig.14 (Cont).(6/8)

```
         NciI  | |   |       RsaI  |                   DpnI
        ScrFI  | |   |      Csp6I| |                BciVI |
       HpaII|  | |   |     Acc65I|| |                BstYI |    BsrFI
       BssKI||  | |   |       BanI|| |                 MboI |   AlwI |
          |||  | |   |           ||| |                    | |       | |
       aaccgggcgcggtcggtaaagtggtaccatttttgaagcgaaggttgtggatctggata
  1081 ---------+---------+---------+---------+---------+---------+ 1140
       ttggcccgcgccagccatttcaccatggtaaaaacttcgcttccaacacctagacctat

c        P  G  A  V  G  K  V  V  P  F  F  E  A  K  V  V  D  L  D  T -

                                                       AvaII
                                                      Sau96I
                                                      SacII|
                                                      AciI||
                                                     BstUI||
                                                    MspA1I||
                                                    AciI |||
                                                   BsaJI |||
  HpaII               MseI             Bst4CI     BtgI |||
      |                  |                  |       |  |||
       ccggcaaaacgctgggcgttaatcagcgtggcgaactgtgtgtccgcggtcctatgatta
  1141 ---------+---------+---------+---------+---------+---------+ 1200
       ggccgttttgcgacccgcaattagtcgcaccgcttgacacacaggcgccaggatactaat

c        G  K  T  L  G  V  N  Q  R  G  E  L  C  V  R  G  P  M  I  M -

                                                                Fnu4HI
                                                                CviJI|
                                                                 TseI|
                           HpaII                              Cac8I ||
     HpaII                BsaWI|                            BstF5I| ||
     BsaWI|               BspEI|       MwoI        BbvI     CviJI|| ||
          ||                  ||          |           |          ||| ||
       tgtccggttatgtaaacaatccggaagcgaccaacgcccttattgacaaggatggctggc
  1201 ---------+---------+---------+---------+---------+---------+ 1260
       acaggccaatacatttgttaggccttcgctggttgcgggaataactgttcctaccgaccg

c        S  G  Y  V  N  N  P  E  A  T  N  A  L  I  D  K  D  G  W  L -

                                               MboII
                                            BsmFI |
     FokI                                  BbsI |  |
     BsmI|                             MboII |  |  |              AciI
  HpyCH4V|                     BsrI BmrI  |  |  |  |     HincII  |
        ||                        |    |  |  |  |  |          |  |
       tgcattctggcgacatcgcttactgggacgaagacgaacacttcttcatcgttgaccgcc
  1261 ---------+---------+---------+---------+---------+---------+ 1320
       acgtaagaccgctgtagcgaatgaccctgcttctgcttgtgaagaagtagcaactggcgg

c        H  S  G  D  I  A  Y  W  D  E  D  E  H  F  F  I  V  D  R  L -

                                                 BcgI       DpnI
                                              AluI |        MboI |
                                             CviJI |       ClaI| |
                                            MspA1I |       TaqI| |
                                         NlaIV   | |      BsrI || |
```

# Fig.14 (Cont). (7/8)

```
                                        CviJI|     | |  HinfI| || |
              MseI        CviJI        HaeIII|     | |   TfiI| || |
          BsmAI  |   Eco57I    |      Sau96I||PvuII  |   AlwI|| || |
                |  |       |   |           |||     | |      ||| || |
     tgaagtctctcattaaatacaaaggctatcaggtggccccagctgaactggaatcgatcc
1321 ---------+---------+---------+---------+---------+---------+ 1380
     acttcagagagtaatttatgtttccgatagtccaccggggtcgacttgaccttagctagg

c     K  S  L  I  K  Y  K  G  Y  Q  V  A  P  A  E  L  E  S  I  L -

                                 BbsI
                                 HgaI
                                MboII
                            Cac8I   |
                            AciI |  |
                           BspMI |  |
                           BstUI |  |
                          Hpy99I |  |
           MboII      TaqI     | |  |      HpaII       HpaII
           MnlI |     FauI |   | |  |     BsaWI|      BsrFI|
     HpyCH4V | |    BcgI | |   | ||MwoI    BspEI|      SgrAI|
           |   | |     | | |   | |  |          ||     BsaHI ||
     tcctgcaacacccaaacatcttcgacgcgggcgtggcaggtcttccggacgatgacgccg
1381 ---------+---------+---------+---------+---------+---------+ 1440
     aggacgttgtgggtttgtagaagctgcgcccgcaccgtccagaaggcctgctactgcggc

c     L  Q  H  P  N  I  F  D  A  G  V  A  G  L  P  D  D  D  A  G -

          AciI
         BsiEI
        Fnu4HI
        CviJI|
       HaeIII|               Bst4CI
         HphI|             BsiHKAI |
        EaeI ||           Bsp1286I |
        EagI ||          TaqI    | |
   HgaI HpaII| ||        AvaI|   | |                        DpnI
      |     || ||        SmlI|   | |                       MboI |
                         XhoI|   | |                          | |
     gtgaacttccggccgccgttgttgttctcgagcacggtaagacgatgacggaaaaagaga
1441 ---------+---------+---------+---------+---------+---------+ 1500
     cacttgaaggccggcggcaacaacaagagctcgtgccattctgctactgcctttttctct

c     E  L  P  A  A  V  V  V  L  E  H  G  K  T  M  T  E  K  E  I -

                                             AciI
                                            BstUI
                                             HhaI
                                          HinP1I |
             BsrI                        Fnu4HI | |
          Hpy99I|             BstUI       AluI| | |
           TaiI ||    BcgI    AciI |     CviJI| | |
        MaeII | || MaeIII BbvI  | |      TseI| | |      BcgI
            | | ||     |     |  | |         || | |        |
     tcgtggattacgtcgccagtcaagtaacaaccgcgaaaaagctgcgcggtggcgttgtgt
1501 ---------+---------+---------+---------+---------+---------+ 1560
     agcacctaatgcagcggtcagttcattgttggcgctttttcgacgcgccaccgcaacaca
```

# Fig.14 (Cont).(8/8)

```
c       V  D  Y  V  A  S  Q  V  T  T  A  K  K  L  R  G  G  V  V  F -

                                                 HgaI        DpnI
                                                TaiI|      BstYI |
                                             BsaAI ||       MboI |
                                            MaeII| ||  BstUI   | |
              RsaI         HpaII        Hpy99I || || AciI |   | |
            Csp6I|       BsrFI|     TaqI     | || || AlwI |   | |
                 ||          ||        |     | || ||     | |   | |
        ttgtggacgaagtaccgaaaggtcttaccggcaaactcgacgcacgtaaaatccgcgaga
   1561 ---------+---------+---------+---------+---------+---------+ 1620
        aacacctgcttcatggctttccagaatggccgtttgagctgcgtgcattttaggcgctct

c       V  D  E  V  P  K  G  L  T  G  K  L  D  A  R  K  I  R  E  I -

            CviJI
           HaeIII
          BslI  |                      MseI
         MseI|  |                  AluI |
       EcoNI||MnlI                CviJI |
           |||  |      AciI     HindIII | |
           |||  |        |          | | |
        tcctcattaaggccaagaagggcggtaagtccaagctttaa
   1621 ---------+---------+---------+---------+- 1661
        aggagtaattccggttcttcccgccattcaggttcgaaatt

c       L  I  K  A  K  K  G  G  K  S  K  L  *  -
```

# Fig.15.

Inactivation of mutants E354I+D357Y and IDRIS (FA) at 50C

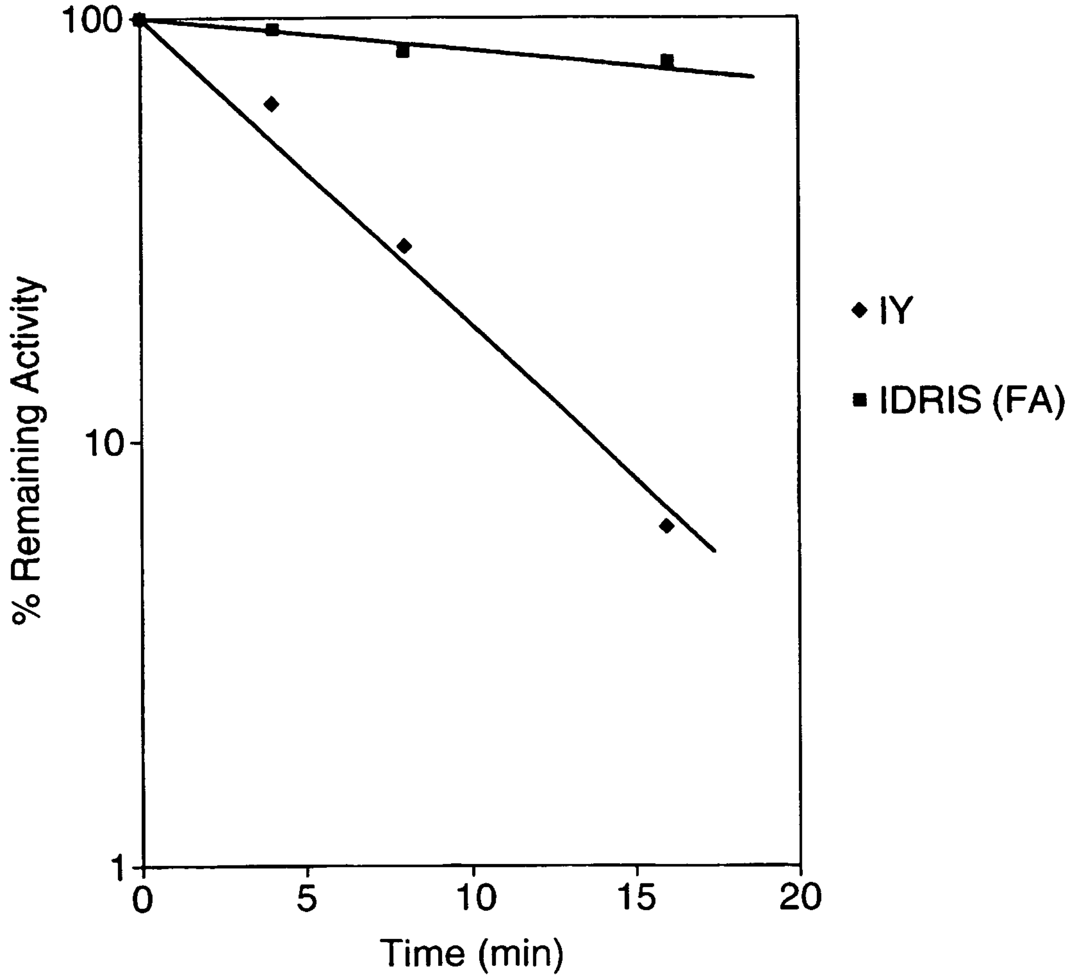

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 680515 B
- WO 9602665 A
- WO 9518853 A
- EP 524448 A
- WO 9525798 A
- GB 2345913 A
- WO 0024878 A
- WO 0120002 A
- WO 9622376 A
- WO 9846729 A
- EP 0524448 A
- GB 9925161 A
- GB 0016744 A


### Non-patent literature cited in the description


- *Biochem.,* 1999, vol. 38, 8271-8279
- **LIPMAN ; PEARSON.** *Science,* 1985, vol. 227, 1435-1441
- **L. YE et al.** *Biochim. Biophys Acta,* 1997, vol. 1339, 39-52
- **SAMBROOK et al.** Molecular Cloning. Cold Spring Harbor Laboratory Press, 1989
- **LIPMAN, D.J. ; PEARSON, W.R.** Rapid and Sensitive Protein Similarity Searches. *Science,* 1985, vol. 227, 1435-1441
- **M. FROMANT et al.** *Anal. Biochem.,* 1995, vol. 224, 347-353
- **F. SANGER et al.** *Proc. Natl. Acad. Sci. U.S.A.,* 1977, vol. 74, 5463-5467
- **E. CONTI et al.** *Structure,* 1996, vol. 4, 287-298
- **A.R ARKIN et al.** *Bio-technology,* 1992, vol. 10, 297-300
- **W,. HUANG et al.** *Anal. Biochem.,* vol. 218, 454-457
- **S. CLIMIE et al.** *J. Biol. Chem.,* 1990, vol. 265, 18776-18779